# EUROPEAN PATENT SPECIFICATION

(11) **EP 1 255 716 B1**
(45) Date of publication and mention of the grant of the patent: **07.01.2009**
(21) Application number: 01908463.1
(22) Date of filing: 16.02.2001
(51) Int. Cl.: C40B 30/04, C40B 40/06, C40B 50/08, C07K 1/04, G01N 33/68

(54) **SEGMENT SYNTHESIS**
SEGMENT-SYNTHESE
SYNTHESE DE SEGMENT

(30) Priority: 16.02.2000 EP 00200536
(43) Date of publication of application: 13.11.2002
(73) Proprietor: PEPSCAN SYSTEMS B.V., 8219 PH Lelystad (NL)
(72) Inventor: PUIJK, Wouter, Cornelis, NL-8243 VZ Lelystad (NL); SLOOTSTRA, Jelle, Wouter, NL-8232 AJ Lelystad (NL); VAN DIJK, Evert, NL-8355 CG Giethoorn (NL)
(74) Representative: Prins, Adrianus Willem
(86) International application number: PCT/NL2001/000131
(87) International publication number: WO 2001/060769

(56) References cited:
- WO-A-98/54577
- DE-A- 19 703 801
- US-A- 5 629 179
- KIM S W ET AL: "Construction of Combinatorial Chemical Libraries Using a Rapid and Efficient Solid Phase Synthesis Based on a Multicomponent Condensation Reaction" TETRAHEDRON LETTERS,NL,ELSEVIER SCIENCE PUBLISHERS, AMSTERDAM, vol. 39, no. 39, 24 September 1998 (1998-09-24), pages 6993-6996, XP004133582 ISSN: 0040-4039
- Y. FENG ET AL.: "Solid-phase Sn2 macrocyclization reactions to form beta-turn mimics" ORGANIC LETTERS., vol. 1, no. 1, 1999, pages 121-124, XP000901551 ACS, WASHINGTON, DC., US ISSN: 1523-7060
- V. SWALI ET AL.: "Solid-phase dendrimer synthesis and the generation of super-high-loading resin beads for combinatorial chemistry" JOURNAL OF ORGANIC CHEMISTRY, vol. 62, no. 15, 1997, pages 4902-4903, XP000901550
- P. A. TEMPEST ET AL.: "solid-phase, parallel syntheses by Ugi multicomponent condensation" ANGEWANDTE CHEMIE. INTERNATIONAL EDITION., vol. 35, no. 6, 1996, pages 640-642, XP000891775 VERLAG CHEMIE. WEINHEIM., DE ISSN: 0570-0833

## Description

The invention relates to the field of molecular recognition or detection of discontinuous or conformational binding sites or epitopes corresponding to or interacting with a binding molecule, in particular in relation to protein-protein or protein-ligand interactions.

Interactions between binding molecules, which in general are biomolecules, and their corresponding ligands, are central to life. Cells often bear or contain receptor molecules that interact or bind with a hormone, a peptide, a drug, an antigen, an effector molecule or with another receptor molecule; enzymes bind with their substrate; antibody molecules bind with an antigen, nucleic acid with protein, and so on. By "interact or bind" it is meant that the binding molecule and ligand approach each other within the range of molecular forces, and may influence each others properties. This approach takes the binding molecule and its ligand through various stages of molecular recognition comprising increasing degrees of intimacy and mutual effect: they bind.

Binding molecules have this binding ability because they comprise distinct binding sites allowing for the recognition of the ligand in question. The ligand, in turn, has a. corresponding binding site, and only when the two binding sites can interact byessentially spatial -- complementarity, the two molecules can bind. Needless to say that, molecules having three dimensions, binding sites are of a three dimensional nature, often one or more surface projections or protuberances of one binding site correspond to one or more pockets or depressions in the other, a three-dimensional lock-and-key arrangement, sometimes in an induced-fit variety.

Sometimes, such a protuberance comprises a single loop of the molecule in question, and it is only this protuberance that essentially forms the binding site. In that case one often terms these binding sites as comprising a linear or continuous binding site, wherein a mere linear part of the molecule in question is in essence responsible for the binding interaction. This terminology in widely used to describe for example antibody-antigen reactions wherein the antigen comprises part of a protein sequence, a linear peptide. One than often speaks about a linear or continuous epitope, whereby the binding site (epitope) of the antigenic molecule is formed by a loop of consecutively bound amino acids. However, similar continuous binding sites (herein epitope and binding site are use interchangeably) can be found with receptor-antigen interactions (such as with a T-cell receptor), with receptor-ligand interactions such as with hormone receptors and agonists or antagonists thereof, with receptor-cytokine interactions or with for example enzyme-substrate or receptor-drug interactions, whereby a linear part of the molecule is recognised as the binding site, and so on. More often, however, such a protuberance or protuberances and depressions comprise various, distinct parts of the molecule in question, and it are the combined parts that essentially form the binding site. Commonly, one names such a binding site comprising distinct parts of the molecule in question a discontinuous or conformational binding site or epitope. For example, binding sites laying on proteins having not only a primary structure (the amino acid sequence of the protein molecule), but also secondary and tertiary structure (the folding of the molecule into alpha-helices or beta-sheets and its overall shape), and sometimes even quaternary structure (the interaction with other protein molecules) may comprise in their essential protuberances or depressions amino acids or short peptide sequences that lay far apart in the primary structure but are folded closely together in the binding site.

Due to the central role binding molecules and their ligands play in life, there is an ever expanding interest in testing for or identification of the nature or characteristics of the binding site. Not only is one interested in the exact nature of the particular interaction between binding molecule and ligand in question, for example in order to replace or supplement binding molecules or ligands when needed; one is also interested in knowing approximating characteristics of the interaction, in order to find or design analogues, agonists, antagonists or other compounds mimicking a binding site or ligand involved.

Versatile and rapid methods to test for or identify continuous epitopes or binding sites are known. Most, if not all nucleic acid detection techniques, and molecular libraries using these, entail hybridisation of an essentially continuous nucleic acid stretch with a complementary nucleic acid strand, be it DNA, RNA or PNA. Little attention has been paid to methods allowing rapid and straightforward identification of discontinuous binding sites of an essentially nucleic acid nature. Although plenty of such sites exist, think only of the lack of understanding surrounding ribosomal binding sites where ribosomal proteins bind to tRNA, of regulatory sites in promotor sequences, of interactions between polymerases and replicases between DNA and RNA, and so on, no molecular libraries exist that provide easy access to such sites. An early work in the peptide field is WO 84/03564, related to a method of detecting or determining antigenically active amino acid sequences or peptides in a protein. This work, providing the so-called Pepscan technology, whereby a plurality of different peptides is synthesised by linking with a peptide bond a first amino acid to a second, and so on, and on a second position in the test format yet another first amino acid is linked to a second, and so on, after which the synthesised peptides are each tested with the binding molecule in question, allows the determination of every continuous antigenic determinant or continuous epitope of importance in a protein or peptide sequence. Pepscan technology taken in a broad sense also provides for the testing for or identification of (albeit linear) peptides essentially identical with, analogous to or mimicking binding sites or ligands of a various nature (mimitopes, Geyssen at al, Mol. Immunol. 23:709-715, 1986).

Pepscan technology allows identification of linear peptide sequences interacting with receptor molecules, enzymes, antibodies, and so on, in a rapid and straightforward fashion, allowing testing a great many peptides for their reactivity with the binding molecule in question with relatively little effort. The order of magnitude of testing capability having been developed with Pepscan technology (e.g. also due to miniaturisation of test formats, see for example WO 93/09872) furthermore allows at random testing of a multiplicity of peptides, leading to automated combinatorial chemistry formats, wherein a great many of binding molecules are being tested in a (if so desired at random) pattern for their reactivity with a molecular library of synthetic peptides representing potential continuous binding sites or ligands, allowing the rapid detection of particularly relevant molecules out of tens of thousands of combinations of molecules tested.

However, for the testing of discontinuous or conformational binding sites to a binding molecule, no formats similar to or as versatile as Pepscan technology exist. Attempts to identify discontinuous epitopes by Pepscan technology are cumbersome. It does in general not suffice to merely extend synthesis of the test peptides by linking more amino acids to the existing peptide, and hoping that some of the thus formed longer peptides will fold in such a way that at least two distinct parts are presented in a discontinuous fashion and are recognised by a binding molecule. Than there is no way of finding out in a rapid and straightforward fashion that the binding is indeed through a discontinuous binding site, it might be that just a longer single loop is responsible for the binding.

Some additional possibilities are provided by testing synthetic peptide sequences that have been designed to comprise two previously identified parts of a binding site, each part in essence being linear and being part of a larger linear peptide. Early work herein was done by Atassi and Zablocki (J. Biol. Chem 252:8784, 1977) who describe that spatially or conformationally contiguous surface residues (which are otherwise distant in sequence) of an antigenic site of egg white lysozyme were linked by peptide bonds into a single peptide which does not exist in lysozyme but attempts to simulate a surface region of it. However, their technique, called surface simulation synthesis, requires the detailed knowledge of the three-dimensional structure of the protein under study and a full chemical identification of the residues constituting the binding site at beforehand, as well as their accurate conformational spacing and directional requirements.

In the same fashion, Dimarchi et al (Science 232:339-641, 1986) describe a 38 to 40 amino acid long synthetic peptide consisting of two previously identified separate peptidyl regions of a virus coat protein. The peptide was synthesised using common peptide synthesis technology (Merrifield et al., Biochemistry 21, 5020, 1982) by adding subsequent amino acids with a peptide bond to an ever growing peptide resulting in a peptide wherein the two peptidyl regions were connected by a diproline spacer presumably functioning as indication of a secondary structural turn, thereby thus providing a two-part epitope or binding site.

However, it is clear that when one already at beforehand has to know the sequence of the (in this case only) two relevant parts, in order to provide the desired discontinuous binding site, it excludes the feasibility to provide (desirably in a random fashion) a whole array of merely potential discontinuous binding sites for large scale testing. Furthermore, a major drawback of these strategies is that again only linear epitopes or dominant binding regions of discontinuous eptitopes can be mimicked adequately. For the complete synthesis of a discontinuous binding site, all the contributing parts have to be arranged in the proper comformation to achieve high-affinity binding, therefore, single parts of discontinuous binding sites have to be linked.

Fifteen years after Dimarchi, Reineke et al (Nature Biotechnology, 17:271-275, 1999) provided a synthetic mimic of a discontinuous binding site on a cytokine and a method to find such a discontinuous binding site that allowed for some flexibility and somewhat larger scale of testing, wherein positionally addressable peptide collections derived from two separate regions of the cytokine were displayed on continuous cellulose membranes, and substituted in the process to find the best binding peptide. After selection of the "best reactors" from each region, these were combined to give rise to another synthetic peptide collection (comprising peptides named duotopes) that again underwent several rounds of substitutions.

Reineke et al hereby provide synthesis of peptide chains comprising duotopes, however, again selected after previous identification of putative constituting parts with Pepscan technology, thereby still not allowing testing discontinuous binding sites in a rapid and straight forward fashion.

However, as indicated before, protein domains or small molecules that mimic binding sites are playing an increasing role in drug discovery, diagnostics and biotechnology. The search for particular molecules that bind to a binding site and mimic, or antagonise the action of a natural ligand has been initiated in many laboratories. As indicated before, attempts to find such structures in synthetic molecular libraries often fail because of the essentially discontinuous nature and spatial complementarity of most binding sites. Thus, for the many more cases where the binding site may essentially be discontinuous improved means and methods to identify these sites are needed, and in particular means and methods are needed that allow testing for discontinuous binding sites whereby said parts need not necessarily first be selected by previous identification as a putative or even only tentative constituting part of the desired discontinuous binding site, but bear only the potentiality of being part of that site by being a molecule with more or less distinct features per se.

The invention provides a method for producing a molecular library comprising providing said library with a plurality of molecules wherein said molecules have essentially been produced by segmental linkage, that is by linking (tri, oligo- or multimeric) nucleic acids or peptides, instead of by sequentially synthesising said molecules which is done traditionally. The invention thus provides a molecular library that, albeit also suited for detecting or screening for continuous binding sites, is now particularly well suited for detecting or screening for discontinuous binding sites, in particular in relation to binding molecule-ligand interactions such as for example protein-protein, protein-nucleic acid, and nucleic acid-nucleic acid interactions.

Said nucleic acids or peptides can of course be selected at random from any set of tri, - or oligonucleotides, or tri-, or oligopeptides, but sometimes it may be preferred to include at least one specific segment in said nucleic acid or peptide, specific in a sense that it has been selected from among known segments or distinct parts of biomolecules, such as parts of genes, proteins, enzymes, nucleic acids or unique fragments thereof, proteins involved in up- or downregulation of translation, t-RNAs, SNRP's, antibodies, antigens, receptors, transport proteins, transcription factors or factors involved in up- or downregulation of transcription, promotor sequences such as but not necessarily restricted to the well known TATA-box elements, repressor sites, operator sites and other control elements, polymerases, replicases, in short, from among known segments or distinct parts of binding molecules known or suspected to be involved in binding via a discontinuous binding site. Such known segments or parts thereof may of course be already known as parts constituting a discontinuous binding site, however, previous identification as such is in essence not necessary, since screening for such sites with a plurality of molecules according to the invention allows rapid and straightforward identification of said constituting segments or parts thereof.

Screening such a library can easily be envisioned when the library's molecules differ only in that constituting segments are chosen in an overlapping fashion, whereby a first segment from a distinct biomolecule is linked to a second, and to a third, and to a fourth segment, and a second is linked to a third, and to a fourth, and so on, if so required until all possible segments of said biomolecule have been linked two-by-two (or three-by-three, or even more) together, which allows for a systematic screening of said biomolecule. However, linking in a overlapping fashion is of course not required, random segment links will provide valuable information about binding sites as well.

The invention thus provides a method for producing a plurality of molecules for identification or detection of a binding site capable of interacting with a binding molecule, and thus for the identification of a molecule as a binding molecule, said method comprising generating at least one of said molecules, preferably a greater part, most preferably essentially all of said molecules, by at least linking a first nucleic acid or peptide to a second nucleic acid or peptide, wherein said first and second nucleic acid or peptide comprise at least a trinucleotide or tripeptide.

Existing libraries, be it of for example nucleic acid (containing a repetitive back-bone of nucleotides, nucleosides or peptide nucleic acid, or combinations of these) or amino acid (containing a repetitive back-bone of amino acids) nature have in general in common that they comprise oligomeric or multimeric molecules, such as stretches of nucleic acids or amino acids, that have been produced by sequentially linking, in a repetitive fashion, one monomer (e.g. a nucleotide or an amino acid) to another, until a (in essence polymeric) molecule of the desired length has been obtained.

Essentially, existing nucleic acid libraries comprise nucleic acids that are synthesised sequentially, by adding one nucleotide or nucleoside at a time to the growing stretch, and existing peptide libraries comprise peptides that are synthesised sequentially, by adding one amino acid at the time to a growing stretch, until the desired length has been reached. With nucleic acids said monomers are essentially selected from a limited set of well known nucleotides, with peptides, said monomers are essentially selected from a well known set of amino acids. Not only naturally occurring monomers are used, synthetic nucleotides, such as peptide nucleic acid (PNA) molecules, or non-naturally occurring amino acids, or even D-amino acids, are routinely used as monomers by which the essentially polymeric molecules are generated or produced, using a method that is essentially conform the sequential synthesis of polymers from monomeric molecules in nature.

The invention provides the recognition that essentially using dimeric or even larger (tri-, oligo-, or multimeric) segments, and thus stepping out of fashion with sequential nucleic acid or protein synthesis as it essentially occurs in nature, offers distinct advantages. It not only provides a faster method to arrive at a molecule composed of various segments, it also provides for fast and efficient shuffling of segments to generate a molecule repertoire for the desired library. Herein, a segment comprising a dimer at least consists of a dimer but can also be for example a trimer, or any other multimer, linking monomers of any nature, as required. Of course, once two segments have been linked, further segments can be linked.

In a preferred embodiment, to speed up further synthesis, or to be able to select distinct desired segments, the invention provides a method wherein said first segment also comprises a dimer, and in a yet even more preferred method, further segments comprise dimers as well. In a preferred embodiment, said dimer comprises a dinucleotide or dipeptide, but of course other dimers can be made also. The invention is further explained in the detailed description where several of the examples relate to libraries comprising molecules wherein each of said segments comprises a peptide, such as a tri-, a penta, an octa-, or nonapeptide; it is however also provided by the invention to use segments of a varied nature, e.g. wherein one comprises a nucleic acid and another comprises a peptide, to better mimic binding sites that are for example found on nucleic acid-protein complexes.

In a preferred embodiment, as for example shown in the examples, the invention provides a method wherein said first peptide is linked by a thioether bond to said second peptide however, the invention is of course not limited thereto. Nucleotide/side segments can for example be covalently linked or ligated by splicing enzymes or ligases, or by overlapping a first segment and the second segment with an in essence relatively short nucleotide strand that is partly complementary to both segments.

The invention thus provides a molecular library allowing testing for, identification, characterisation or detection of a continuous or discontinuous binding site capable of interacting with a binding molecule, said library having been provided with a plurality of molecules, each molecule of said molecules preferably comprising at least one first nucleic acid or peptide linked to a second nucleic acid or peptide wherein at least said second nucleic acid or peptide previously existed as dimer or multimer. Preferably, each nucleic acid or peptide or part thereof having the capacity of being a potential single part of a discontinuous binding site, preferably wherein each of at least a first and a second nucleic acid or peptide or part thereof represents a potential single part of a discontinuous binding site. Such a library can for example exist of a synthetic molecular library made by chemical linking of segments.

Herewith the invention provides synthesis of molecules comprising separate segments potentially representing at least two distinct parts of a discontinuous binding site, said parts not necessarily first being selected after previous identification of potential constituting parts, thereby allowing testing for discontinuous binding sites in a rapid and straight forward fashion.

The invention thus now allows identifying discontinuous binding sites of receptor molecules that interact or bind at that contact site with a hormone, a peptide, a drug, an antigen, an effector molecule or with another receptor molecule, of enzymes that bind with their substrate, of antibody molecules that bind with a binding site on an antigen, nucleic acid that binds with protein, and so on. In one embodiment of the invention, all segments comprise a peptide, said segments preferably linked by a stable (non-naturally) occurring non-peptide bond or linker. In this way a plurality of different peptides is synthesised by linking a first peptide segment to a second, and so on, and on a second position in the test or library format yet another first peptide segment is linked to a second, and so on, after which the synthesised peptides are each tested with the binding molecule in question, allowing the determination of a discontinuous antigenic determinant or discontinuous epitope of importance in a protein or peptide sequence. Said peptide segment comprises at least 2 amino acids, and can in principle be as long as desired, e.g. containing a hundred amino acids or even more. In preferred practice, said peptide segment comprises from 3 to 30, preferably from 4 to 20, even more preferably from 5 or 6 to 12 to 15 amino acids, such as 9 or 12 amino acids. Separate segments of course do not necessarily have to be of equal length.

Furthermore, peptide segments to be linked together can be selected at random, or under guidance of (a) known protein or peptide sequence(s). Selection at random provides a random library according to the invention. Selection from known protein or peptide sequences is for example useful when it is desired to find out whether a discontinuous binding site is composed of distinct sites or parts present at distinct proteins or peptides, for example in a protein complex to which a particular binding molecule can bind. Selection of various peptide segments from one known protein or peptide sequence is useful when it is desired to find out whether a discontinuous binding site is composed of distinct sites or parts present at one protein or peptide, for example at a folded protein to which a particular binding molecule can bind. Selection of peptide segments can be done by selecting overlapping peptides from such a known sequence. Overlapping peptides can have for example all but one or two amino acids in common, preferably overlapping in a contiguous fashion, or can overlap with only one or several amino acids. For a quick scan for discontinuous binding sites on a known protein, it is for example useful to select nonapeptide segments from said protein sequence, of which one has for example a 5-amino acid long overlap with another peptide segment. Equally useful, however, is to select tripeptide segments from said sequence having an overlap of only one amino acid, and use three, or even more segments in constructing the putative binding site molecule to which the to be tested binding molecule can bind.

Of course, such selection strategies are equally applicable to segments of a different nature, nucleic acid segments, comprising a certain number of nucleotides, such as 5, 7, 9, and so on, can be selected from known nucleic acid sequences comprising sought after discontinuous binding sites, each segment selected from a certain position in said known nucleic acid sequence, if desired also in a overlapping fashion. Said nucleic acid segment comprises at least 2 nucleotides (be it DNA, RNA or PNA, or functional equivalents thereof), and can in principle be as long as desired, e.g. containing a hundred nucleotides or even more. In preferred practice, said nucleic segment comprises from 3 to 30, preferably from 4 to 20, even more preferably from 5 or 6 to 12 to 15 nucleotides, such as 9 or 12 nucleotides. Separate segments of course do not necessarily have to be of equal length, and, as said before, can even be of a different nature, e.g. peptide with DNA.

Herein a peptide bond is being defined as an amide bond between an alpha-amino group of one amino acid or peptide and an alpha-carboxyl group of another amino acid or peptide. A non-peptide bond comprises any other amide bond or non-amide bonds. The links or bonds can be formed using many combinations of strategies of for example peptide or nucleotide chemistry and selective ligation reactions as known in the art. Ligation chemistry has been published, for instance, by groups of Kent (Ph.E.Dawson et al., Synthesis of Proteins by Native Chemical Ligation, Science 266 (1994) 776-779), Tam (J.P.Tam et al., Peptide Synthesis using Unprotected Peptides through Orthogonal Coupling Methods, Proc. Natl. Acad. Sci. USA 92 (1995) 12485-12489; C.F.Liu et al, Orthogonal Ligation of Unprotected Peptide Segments through Pseudoproline Formation for the Synthesis of HIV-1 Protease Analogs, J.Am.Chem.Soc. 118 (1996) 307-312; L.Zhang & J.P.Tam Thiazolidone Formation as a General and Site-specific Conjugation Method for Synthetic Peptides and Proteins, Analytical Biochemistry 233 (1996) 87-93), and Mutter (G.Tuchscherer & M.Mutter, Protein Design as a Challenge for Peptide Chemists, J.Peptide Science 1 (1995) 3-10; S.E.Cervigni et al, Template-assisted Protein Design: Chimeric TASP by Chemoselective Ligation, Peptides: Chemistry, Structure and Biology, P.T.P Kaumaya & R.S. Hodges eds, Mayflower (1996) 555-557).

Possible strategies for the formation of links as preferably provided by the invention are for example are:
1. Said link with a segment or segments is formed using a homo- or hetero-bifunctional linking agent (S.S.Wong: Chemistry of Protein Conjugation and CrossLinking, CRC Press Inc, Boca Raton, Florida USA 1991). In this construction a reactive group in one segment is used to react with one part of the bifunctional linking agent, thus facilitating the second part of the linking agent to react with a reactive group from a second segment. For instance, a linker like MBS (m-maleinimidobenzoic acid N-hydroxysuccinimide ester) can be used to react via its active ester (succinimide) with an amino group of one segment and via its maleinimide group with a free thiol group from a second segment. In this strategy preferably no other free amino- or thiol groups should be present in the first segment and preferably no other free thiol groups are present in the second segment. In order to accomplish this, the amino or thiol groups that should be involved in the reaction can be deprotected selectively, for instance, by using a side chain protecting group that can be cleaved by a mild reagent like 1% trifluoroacetic acid, which leaves other side chain protecting groups intact.
2. Said link is formed by introduction of a modified amino acid in the synthesis of one or more segments. Amino acids can be modified, for instance, by introduction of a special group at the side-chain or at the alpha-amino group. A modification at the alpha-amino group leads to an amide or backbone modified peptide (see fort example Gillon et al., Biopolymers, 31:745-750, 1991). For instance, this group can be a maleinimido group at the side chain amino group of lysine. At the end of the peptide synthesis this group will react fast and selective with a thiol group of a second segment. Tam et al. (PNAS 92:12485-12489, 1995) described a synthesis of a peptide with a lysine residue that was modified in the side chain with a protected serine residue. After deprotection and selective oxidation using periodate, the alpha-amino, beta-hydroxy function of the serine was converted into an aldehyde function that could be ligated selectively with another thiol-bearing segment. Also peptide backbone links, via groups attached to the amide groups of the peptide, can be used to link segments (Bitan et al., J. Chem. Soc. Perkin Trans.1:1501-1510, 1997; Bitan and Gilon, Tetrahedon, 51:10513-10522, 1995; Kaljuste and Unden, Int. J. Pept. Prot. Res. 43:505-511, 1994).
3. Yet another way to form said link is to synthesise a segment, such as a peptide, with a modified N-terminus. For instance, an N-terminal alpha-haloacetamido group can be introduced at the end of the synthesis. This group reacts fast and selectively with a second segment, i.e. another peptide, which contains a thiol group. For instance, the first segment is synthesised with an N-terminal bromoacetamide and the second segment with a cysteine. Although most alpha-haloacetamide groups, like chloro-, bromo-, or iodoacetamide, will react with thiol groups, in those cases where speedy assembling is required, the bromoacetamide group is preferred because of its ease of introduction and fast and selective reaction with thiol groups.

Furthermore, the invention provides the possibility to address the link in every position of the first and/or the second or consecutive segment. For instance, for peptide segments sets of peptides are synthesised in which a cysteine or a side-chain modified lysine, both amino acid residues being able to ligate selectively with another segment, shifts from the N-terminal amino acid position one by one to the C-terminal amino acid position. Combinations of these possibilities will again lead to new libraries as provided by the invention.

In a preferred embodiment, the invention provides a plurality of molecules wherein said molecules are positionally or spatially addressable, e.g. in an array fashion, if desired aided by computer directed localisation and/or recognition of a specific molecule or set of molecules within the dimensions (e.g. plane or surface) of the support of the plurality of molecules used. In an array, said molecules are for example addressable by their positions in a grid or matrix.

A preferred embodiment of the invention further allows upscaling of the synthesis concerning the number of constructs on for example a solid support per cm². To facilitate generation of a great many possible constructs, containing for example molecules comprising at least two peptide segments of a protein, many thousands of peptide constructs are made. For instance, when all constructs, in which both segments are for instance 12 amino acids long, are derived from a small protein with a length of 100 amino acid residues are needed, already 89 x 89 = 7921 peptide constructs are made, if the segments are only linked, for instance, via the C-terminus of the first segment and the N-terminus of the second segment using only one type of link. For a protein with a length of 1000 amino acid residues at least 989 x 989 = 978121 constructs are made. For efficient ELISA testing of these numbers of constructs, high construct densities on the solid support are preferred. High densities of constructs on a solid support are provided by the invention, wherein for instance, (a layer of) a first segment with a bromoacetamide group at the N-terminus is synthesised on a surface of, for instance, 1 cm². On this peptide-functionalised surface of the support a set of, for instance, 10, preferably 50, preferably 100, or more second peptide segments containing a free thiol group are spotted or gridded, in a positionally or spatially addressable way, giving, after coupling, so many different peptide constructs. Preferably, said support is provided with a surface wherein patches or pixels are interspersed within areas that are materially distinct from said pixels, a so-called pixel array. In particular, the invention provides a support (herein also called a pixel array) wherein the support surface material is of a varied or discontinuous nature as regards to hydrophilicity. In such a support for a high-density micro-array as provided herein, patches or pixels of relative hydrophilicity are preferably interspersed with areas of relative hydrophobicity. Of course there need not be a sharp border between patches and the surrounding area, it is sufficient when distinct material differences or discontinuities exist between the centre of a patch and the middle line of a surrounding area, whereby there is a more or less gradual material change in between. Patches and surrounding areas may be in strict matrix or grid format, but this is not necessary. Patches are in general somewhat, but preferably at least one or two dimensions smaller than the size of the circumference of the positioned droplets or spots of first member molecules that in a later phase will be provided to the support surface, that is preferably at least 3-5, and more preferably at least 10-20 of such e.g. hydrophilic patches fit within the circumference of a later spotted solution of a first member, be it nucleic acid or peptide or any other (bio-)molecule or combination thereof. Of course, a one-to-one fit of patch to droplet or spot is also feasible, even when the patch is larger than a spot, but not necessary and neither is it necessary to apply or provide for the patches in an overly regular pattern. When a droplet or spot is provided, the interspersed hydrophobic character of the support surface will limit the diffusion of any aqueous solution, and thus also, again in a later phase, the diffusion of a solution of an optically detectable substrate (be it as precipitate or as solution) formed after the enzymatic reaction that took place where a first member is bound to a second member of a binding pair, whereby the presence of the relatively hydrophilic patch or patches within said droplet or spot circumference allows said substrate to be positioned or detectable at all.

Spotting can, for instance, be done using piezo drop-on-demand technology, or by using miniature solenoid valves. Gridding can, for instance, be done using a set of individual needles that pick up sub-microliter amounts of segment solution from a microtiter plate, containing solutions comprising the second segments. After the linking reaction, subsequent deprotection and extensive washing of the support to remove uncoupled peptide gives at least a peptide construct density as large as 10 to 50, or even 100 to 200, or up to 50 to 1000 spots per cm². This density allows to screen a great many possible peptide constructs of said proteins for binding with an antibody. For example: in a preferred embodiment 20000 to 100.000 constructs are made on 1000 cm², typically the surface is than screened for binding in ELISA with 100 ml of antibody solution, containing 1 - 10 µg of antibody/ml. For example, indirect or direct fluorescence detection allocates antibody binding constructs. Direct fluorescence detection with confocal scanning detection methods for example allows antibody detection on spots generated with droplets peptide-solution in the sub-nanoliter range, making even higher construct densities feasible. Of course, nucleic acid libraries can be made in a similar fashion.

Furthermore, the invention provides a solid support, preferably a discontinuous matrix array support as explained below, comprising a plurality of molecules according to the invention, said solid support allowing presentation of a potential discontinuous or conformational binding site or epitope to a binding molecule, said solid support having been provided with a plurality of molecules, each molecule of said molecules being a possible representative of said binding site or epitope and for example comprising at least one first peptide or nucleotide covalently linked by a spacer to a second peptide or nucleotide, said spacer comprising at least one non-peptide linkage. In a preferred embodiment, said solid support comprises at least a spot or dot (e.g. putative binding site or peptide construct) density as large as 10, 20, or 50, or even 100, 200, or up to 500 or even 1000 spots per cm², preferably wherein said spots or dots are positionally or spatially addressable.

The invention further provides a method to screen for, i.e. test, identify, characterise or detect a discontinuous binding site capable of interacting with a binding molecule, comprising screening a plurality of molecules as provided by the invention with binding molecules, such as there are for instance antibodies, soluble receptors, which contain a Fc-tail or a tag for detection, receptors on cells, biotinylated molecules or fluorescent molecules. In particular, the invention provides a method to screen for a discontinuous binding site capable of interacting with a binding molecule, comprising screening a library according to the invention with at least one binding molecule and detecting binding between a member of said library and said binding molecule. In a preferred embodiment, said binding is detected immunologically, for example by ELISA techniques.

Furthermore, the invention combines the advantages of high density arraying (testing a lot of binding events in one go) and enzyme-linked assays (very sensitive) allowing to detect more discontinuous binding sites more rapidly. Micro-array systems are provided herein that allow to work with enzyme-linked assays to detect the molecule of interest on high-density supports. Such testing high densities of constructs on a solid support in a enzyme-linked assay is provided by the invention, wherein for instance a first member is provided to or synthesised on a surface of the support in a density of, for instance, 10 or preferably 50, but more advantageously preferably 100, or more, such 200-500 or even 1000 spots per square centimeter. Said first binding pair members are for example spotted or gridded, in a positionally or spatially addressable way, giving so many different constructs on the support with which a second member or binding molecule can react. Of course, spots can overlap, as long as the constituting collection of first member molecules are spatially addressable and distinct. Spotting can, for instance, be done using piezo drop-on-demand technology, or by using miniature solenoid valves. Gridding can, for instance, be done using a set of individual needles that pick up sub-microliter amounts of segment solution from a microtiter plate, containing solutions comprising the first members. When testing peptides, after the linking reaction, subsequent deprotection and extensive washing of the support to remove uncoupled peptide gives at least a peptide construct density as large as 25 to 50, or even 100 to 200, or up to 500 to 1000 spots per cm². This density allows to screen a great many possible peptide constructs of said proteins for binding with an antibody. For example: in a preferred embodiment 25000 to 100.000 constructs are made on 1000 cm², typically the surface is than screened for binding in enzyme-linked assay-be it directly or indirectlywherein a fluorescent substrate is generated with 100 ml of enzyme-labelled probe solution, containing 1 - 10 µg of probe/ml and subsequent development of an optically detectable substrate with established techniques. For example, indirect or direct fluorescence detection allocates antibody binding constructs. Direct fluorescence detection with confocal scanning detection methods for example allows antibody detection on spots generated with droplets peptide-solution in the sub-nanoliter range, making even higher construct densities feasible. Of course, nucleic acid libraries can be made in a similar fashion, using enzyme-labelled nucleic acid probes.

Furthermore, the invention provides a support for a micro-array suitable for testing binding of a first member molecule, wherein said first member is provided by segment synthesis, within an array or library of tentative first member binding molecules with a second member binding molecule said support provided with a surface wherein patches are interspersed within areas that are materially distinct from said patches. In particular, the invention provides a support (herein also called a discontinuous matrix array) wherein the support surface material is of a varied or discontinuous nature as regards to hydrophilicity. In such a support for a high-density micro-array as provided herein, patches of relative hydrophilicity are preferably interspersed with areas of relative hydrophobicity. Of course there need not be a sharp border between patches and the surrounding area, it is sufficient when distinct material differences or discontinuities exist between the centre of a patch and the middle line of a surrounding area, whereby there is a more or less gradual material change in between. Patches and surrounding areas may be in strict matrix or grid format, but this is not necessary. Patches are in general somewhat, but preferably at least one or two dimensions smaller than the size of the circumference of the positioned droplets or spots of first member molecules that in a later phase will be provided to the support surface, that is preferably at least 3-5, and more preferably at least 10-20 of such e.g. hydrophilic patches fit within the circumference of a later spotted solution of a first member, be it nucleic acid or peptide or any other (bio-)molecule or combination thereof. Of course, a one-to-one fit of patch to droplet or spot is also feasible, even when the patch is larger than a spot, but not necessary and neither is it necessary to apply or provide for the patches in an overly regular pattern. When a droplet or spot is provided, the interspersed hydrophobic character of the support surface will limit the diffusion of any aqueous solution, and thus also, again in a later phase, the diffusion of a solution of an optically detectable substrate (be it as precipitate or as solution) formed after the enzymatic reaction that took place where a first member is bound to a second member of a binding pair, whereby the presence of the relatively hydrophilic patch or patches within said droplet or spot circumference allows said substrate to be positioned or detectable at all. The preferred patches as provided herein may also be described as pixels within the spot where finally the optically detectable or fluorescent substrate will be located. Of course, if so desired patches may be hydrophobic where the surrounding area is relatively hydrophilic, when for example solutions or (optically detectable) markers are tested of a more hydrophobic nature.

In a preferred embodiment, said support as provided herein comprises at least a spot or dot (e.g. a collection of first member molecules such as a nucleic acid or peptide construct) density as large as 25 or 50, or even 100, 200, or up to 500 or even 1000 spots per cm² preferably wherein said spots or dots are positionally or spatially addressable, each of said spot or dot covering at least one patch, but preferably from 3-5, or even from 5-15 or more patches or pixels.

Hydrophilic patch size can be modified by selecting the appropriate support material, such as polyethylene or polypropylene or another relatively hydrophobic plastic material, to begin with, or by providing it with patches in the desired size, e.g. by utilizing print technology. Below, a support surface is produced from a relatively hydrophobic polypropylene surface upon which grafts are provided that form the relatively hydrophilic patches. Preferred is to make the grafts with polyacrylic acid, which has an excellently suitable hydrophilic nature. Patch size can be influenced by selecting the appropriate roughness of a polyethylene or polypropylene starting material, said roughness can also be modulated by sanding or polishing, or by any other mechanical (printing) or chemical (etching) method to modulated a surface on which the hydrophilic patches are to be generated. Of course, the smaller the hydrophilic patch size is, the smaller the droplets to be applied can be, preferably up to the size where at least one patch falls within the circumference of the droplets applied.
The invention also provides a method for determining binding of a first member molecule within an library of tentative first member binding molecules with a second member binding molecule comprising use of a support according to the invention, in particular a method comprising providing said support with spots comprising said tentative first member binding molecules, providing a second member binding molecule and detecting binding of a first member molecule with said second member binding molecule.

Preferably, said binding is detected with an optically detectable marker for example wherein said marker comprises a fluorophore, directly or indirectly labelled to a probe such as a nucleic acid or antibody, thus allowing a support according to the invention to be used in any type of micro-array; prevention of diffusion is always welcome to avoid or circumvent problems such as signal overload, however, in a preferred embodiment, the invention provided a method wherein binding pairs are detected via enzyme-linked-assay techniques, where otherwise diffusion or leakage would be much harder to overcome, the further advantage being that enzymetic detection is much more sensitive, thereby allowing to include less copies of tentative first member molecules to be spotted in one spot, thus in general decreasing spot-size, thus allowing to increase spot density, without having to give in on sensitivity. Enzymatic detection can be up to 10-1000 times more sensitive as detection of directly labelled probes.

Suitable enzyme-substrate combinations and methods for use in a method according to the invention are for example found with US4931223 wherein processes are disclosed in which light of different wavelengths is simultaneously released from two or more enzymatically decomposable chemiluminescent 1,2-dioxetane compounds, said compounds being configured, by means of the inclusion of a different light emitting fluorophore in each of them, to each emit light of said different wavelengths, by decomposing each of said compounds by means of a different enzyme. Also, Bronstein et al. BioTechniques 12 #5 (May 1992) pp. 748-753 "Improved Chemiluminescent Western Blotting Procedure" suggests an assay method in which a member of a specific binding pair is detected by means of an optically detectable reaction which includes the reaction, with an enzyme, of a dioxetane so that the enzyme cleaves an enzyme-cleavable group from the dioxetane to form a negatively charged substituent bonded to the dioxetane, the negatively charged substituent causing the dioxetane to decompose to form a luminescent substance. Cano et al. J. App. Bacteriology 72 (1992)provided an example of nucleic acid hybridization with a fluorescent alkaline phosphatase substrate, which advantagously can be used in the invention as well, and Evangelista et al. Anal. Biochem. 203 (1992) teaches alkyl-and aryl-substituted salicyl phosphates as detection reagents in enzyme-amplified fluorescence DNA hybridization assays. In the detailed description herein use is made of a fluorescent substrate for alkaline phosphatase-based detection of protein blots, for use with fluorescence scanning equipment such as Molecular Dynamics FluorImager or Storm instruments, generally known as Vistra ECF and generally only deemed suitable for use in Western blotting, dot and slot blotting applications. The enzymatic reaction of alkaline phosphatase dephosphorylates said ECF substrate to produce a fluorescent product which is, as shown herein, detectable in a method according to the invention. However, not only alkaline phosphatase detection based is provided herein, the invention also provides a method according to the invention wherein a substrate for evaluating glycosidic enzymes comprising a fluorescein derivative such as known from US5208148 is used, which bears a lypophillic character and therefor will preferably reside in hydrofobic areas of the surface.

Furthermore, the invention provides a synthetic molecule comprising a binding site(i.e. located on the detected first member molecule or derivatives thereof) or a binding molecule comprising a binding site identifiable or obtainable by a method according to the invention. Furthermore, use of a support or a method according to the invention for identifying or obtaining a synthetic molecule comprising a binding site or for identifying or obtaining a binding molecule capable of binding to a binding site is provided and the use of such an obtained molecule for interfering with or effecting binding to a binding molecule.

By detecting binding to a specific member of said library, the invention provides said member, a synthetic molecule comprising a discontinuous binding site identifiable or identified or obtainable or obtained by a method according to the invention. Thus the invention provides use of a library according to the invention, use of a solid support according to the invention, or use of a method according to the invention for identifying or obtaining a synthetic molecule comprising a discontinuous binding site or a binding molecule capable of binding therewith. Because now discontinuous binding sites are provided, such a synthetic molecule can advantageously be used in vitro or in vivo for finding a binding molecule, and for effecting and/or affecting binding to a binding molecule, for example to interact or bind with receptor molecules that normally interact or bind with a hormone, a peptide, a drug, an antigen, with an effector molecule, with an agonist, with an antagonist, or with another receptor molecule; with enzymes that normally bind with their substrate; with antibody molecules, with nucleic acid, with protein, in short, with biomolecules. The invention is further explained in the detailed description without limiting the invention.

### Figure legends

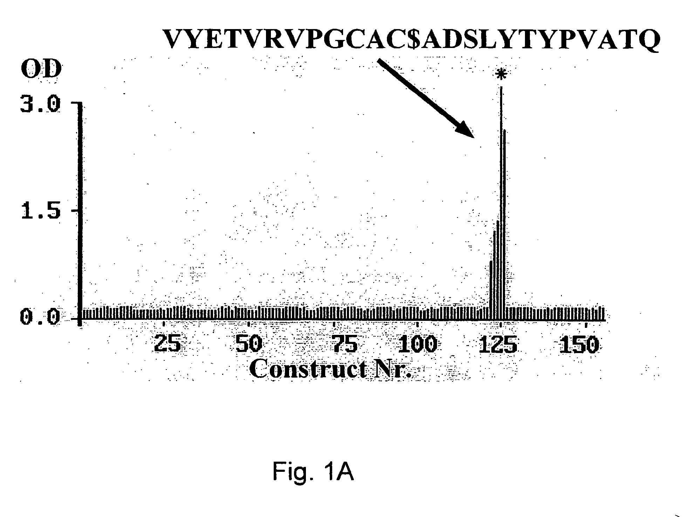

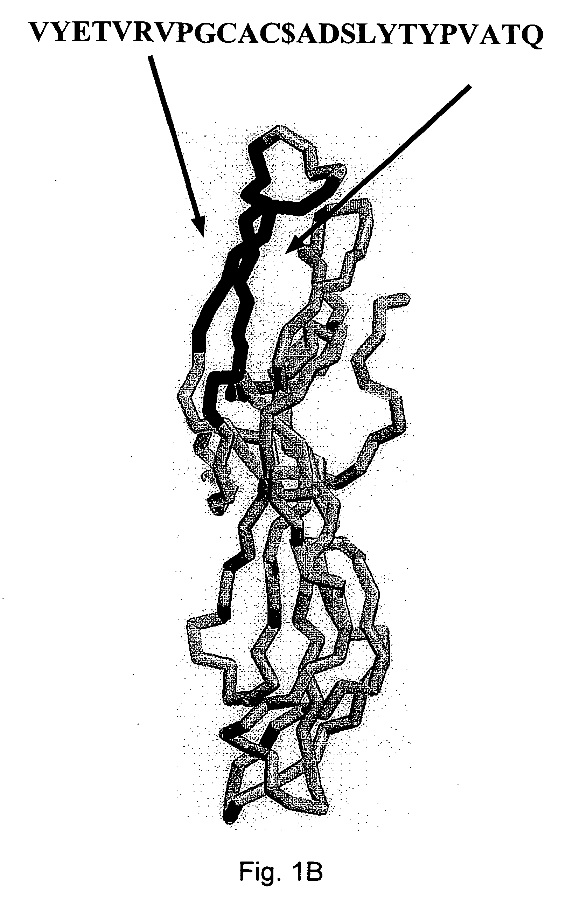

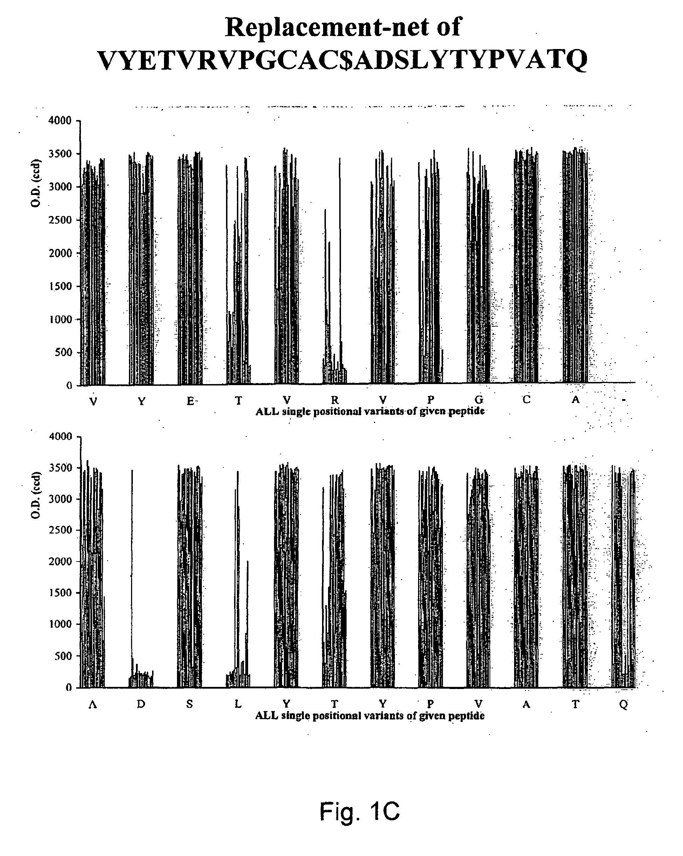

Fig. 1. A) ELISA results of a library of constructs of a protein, synthesised in 3 µl wells of a 455 wells microtiter plate tested against a protein specific monoclonal antibody (monoclonal antibody-01) which binds human Follicle stimulating hormone (hFSH). Construct 1: sequence[1-11]-Cys coupled to bromoacetamide-sequence[14-25]; construct 2: sequence[2-12]-Cys coupled to bromoacetamide-sequence[15-26]; and so on. The reacting peptide is shown. It is part of hFSH as illustrated in Fig. 1B. B) Three dimensional model of hFSH. The identified epitope is shown in black. C) replacement-analysis of the identified epitope. The essential amino acids are part of both parts of the peptides.

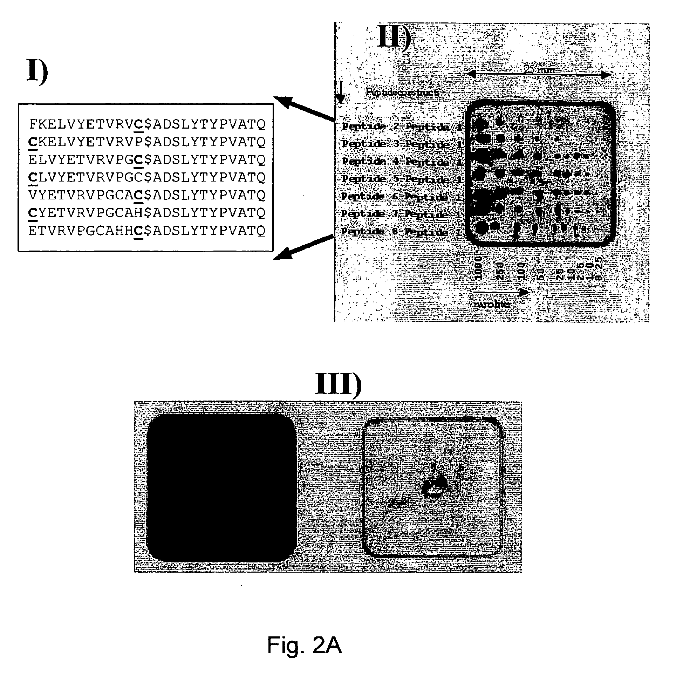

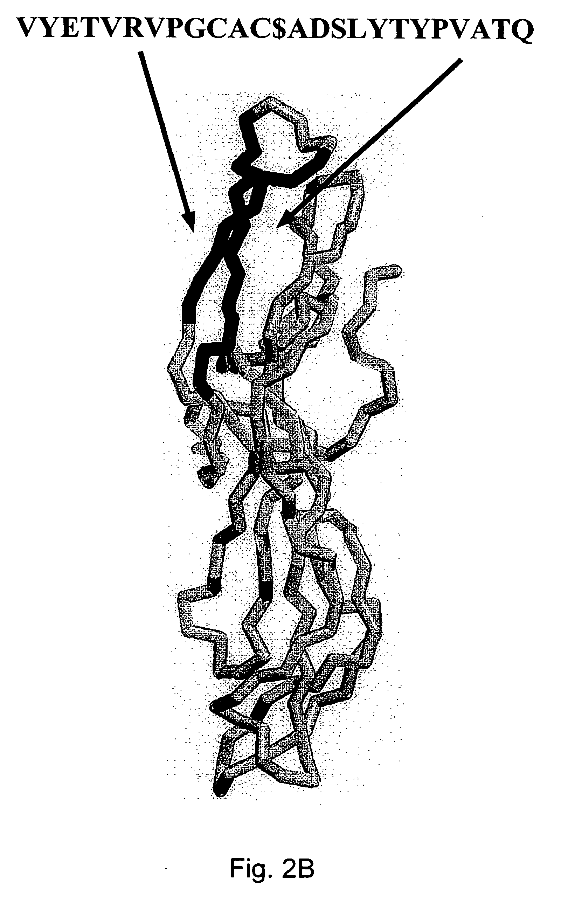

Fig. 2. A) Microturisation of spots of peptide constructs. Constructs were tested against the same monoclonal antibody-01 as was tested in figure 1. Binding was made visible using indirect fluorescence detection. Peptide 1, sequence [139 -150] with an N-terminal bromoacetamide, was synthesised on the complete surface. Peptide construct 6 - 1 is the same as construct 125 in figure 1. Peptides 2 up to 8, containing a cysteine residue, were spotted in different volumes ranging from 1 µl to 0.25 nl using piezo drop-on-demand technology. In I the sequences are shown; in II the spots are shown; in III the controls are shown, on the left a test with monoclonal antibody-03 that recognizes the peptide ADSLYTYPVATQ which is present on the whole square, on the right monoclonal antibody-01 which does not recognize ADSLYTYPVATQ but requires the longer peptides as shown by the spots in I. B) Three dimensional model of hFSH. The identified epitope is shown in black.

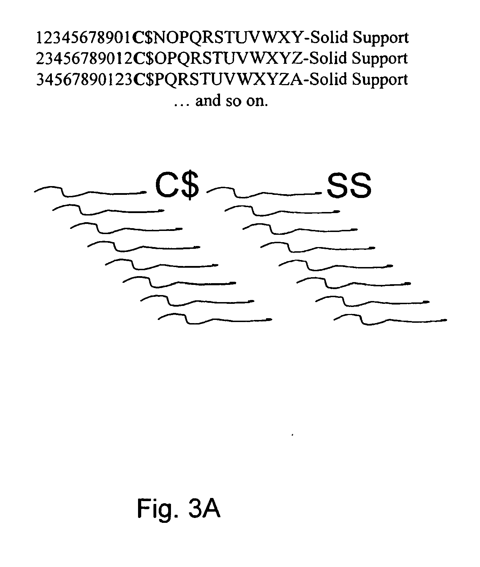

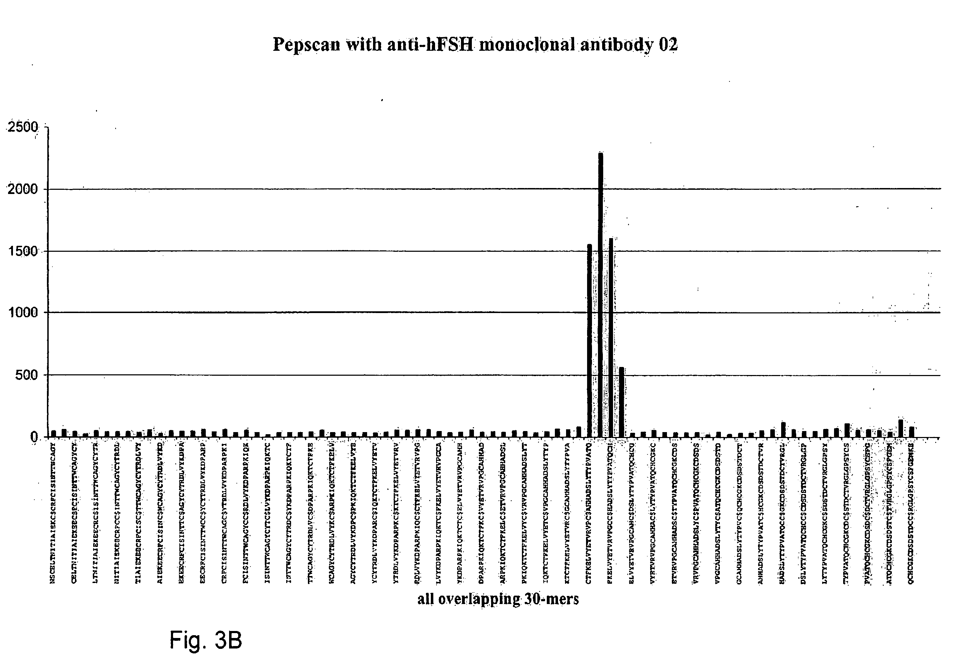

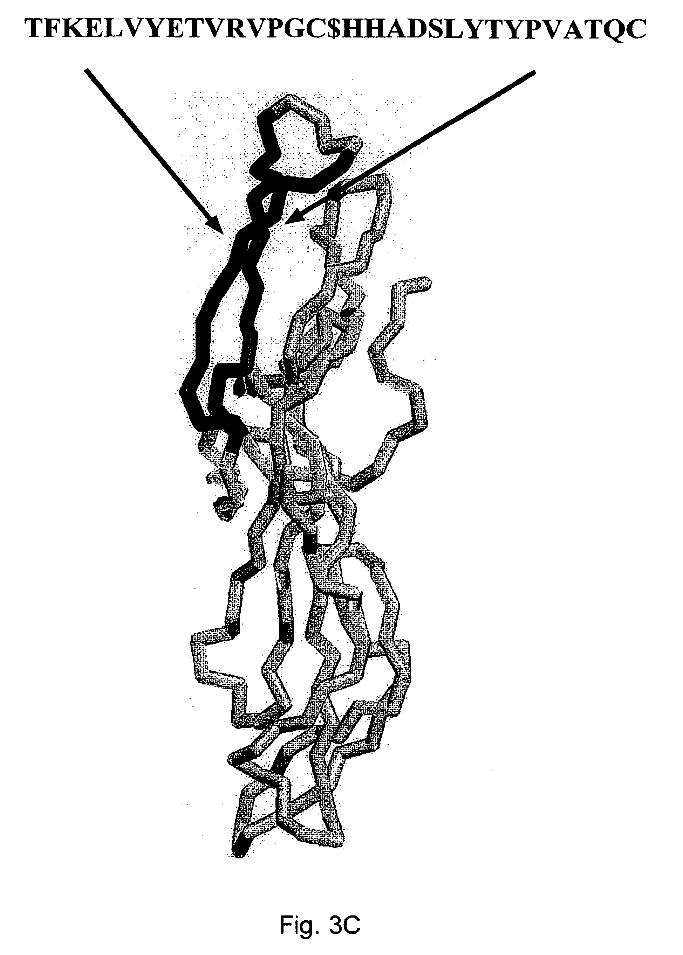

Fig. 3. A) Schematic presentation of a "standard" 24-mer scan in creditcard minicards. 12345678901 (building block 2) and NOPQRSTUVWXY (building block 1) represent successive sequences derived from a protein. Both building blocks are linked via a thioether bridge, formed by reaction of a free thiol function of a C-terminal cysteine residue (C) in building block 2 and a bromoacetamide group ($) at the N-terminus of building block 1. In this scan both sequences are shifted simultanuously by steps of one amino acid residue through the complete protein sequence to obtain the complete library. SS = Solid Support. B) Working example with anti-hFSH monoclonal antibody-02 C) Three dimensional model of hFSH. The identified epitope is shown in black.

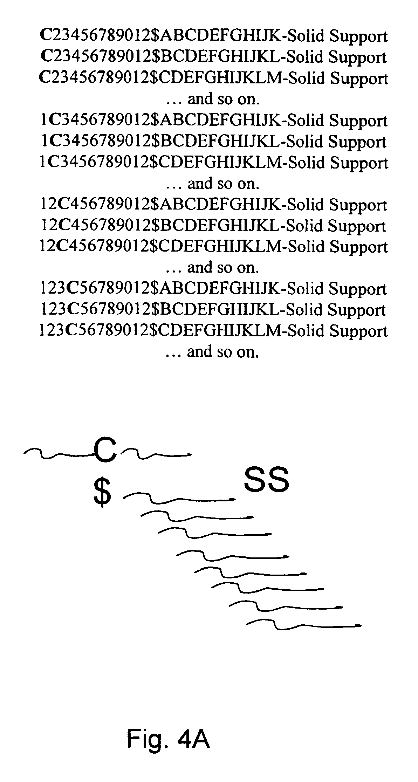

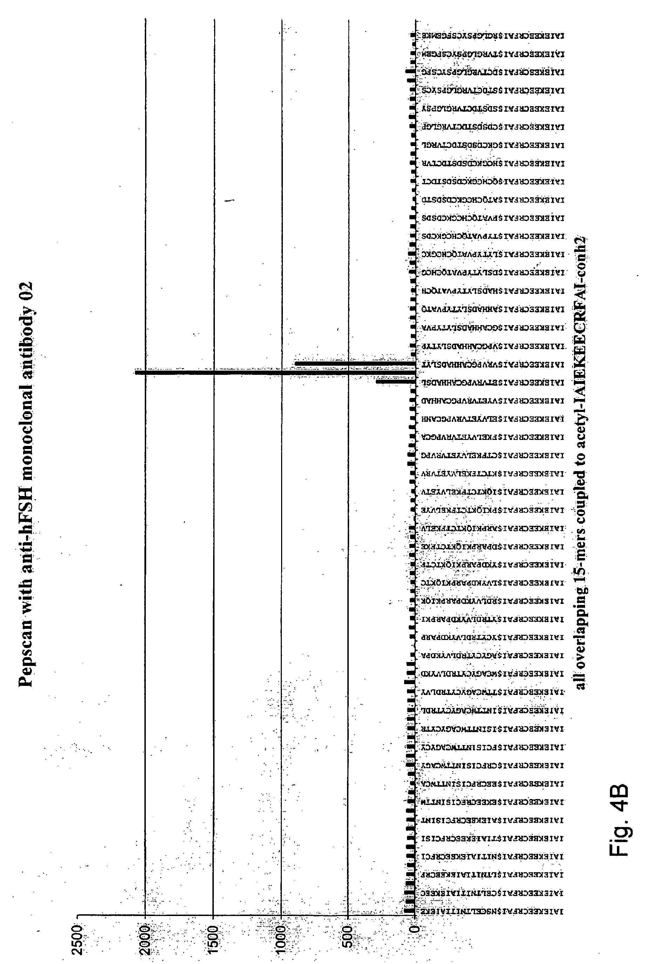

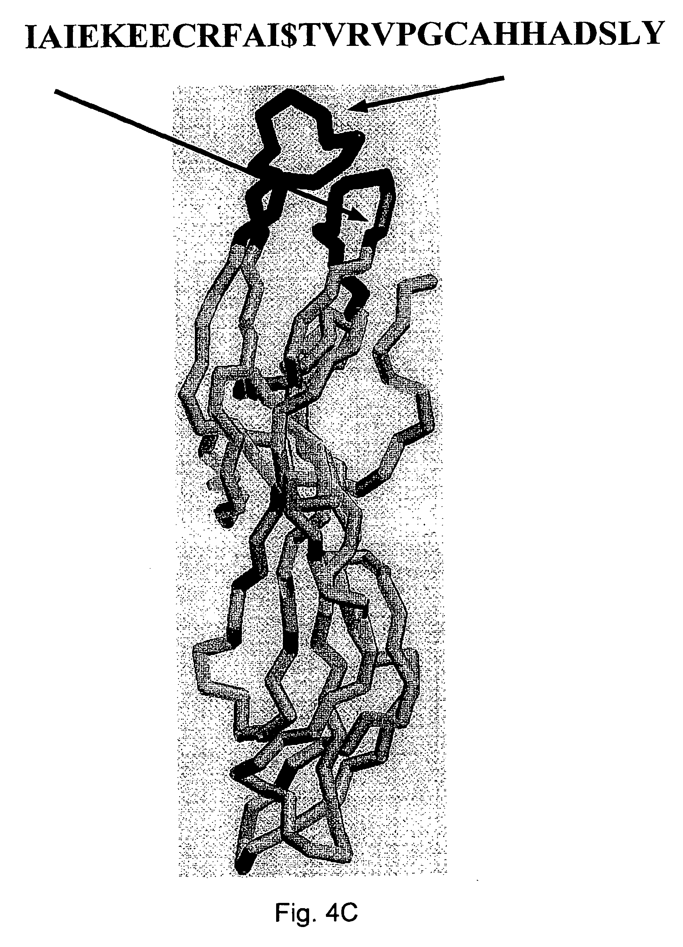

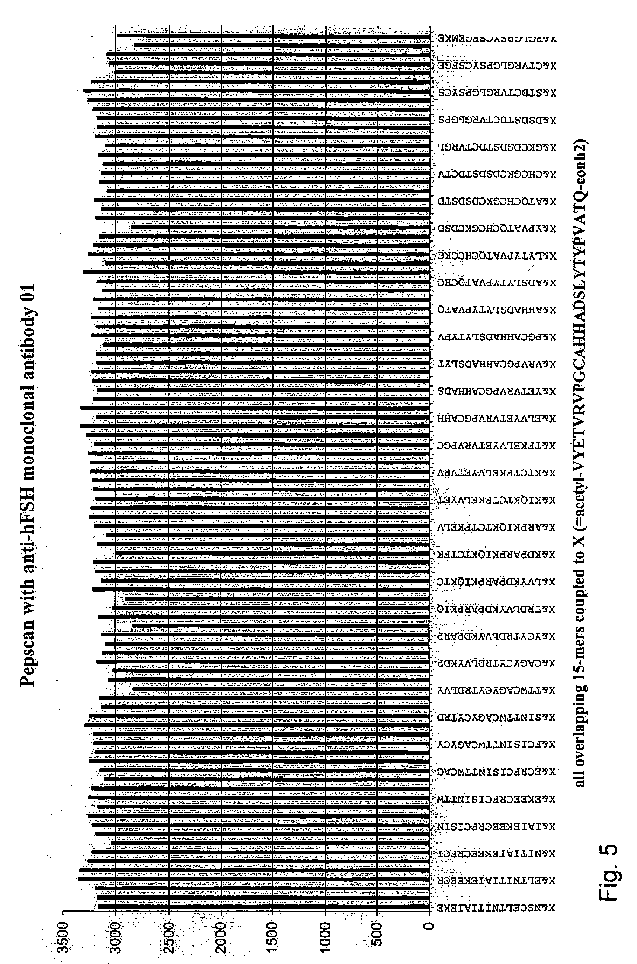

Fig. 4. A) Schematic presentation of a positional complete matrix scan. This scan is similar to the scans shown in fig. 5, however, no cysteine residue was added to one of the termini of the second building block, but instead each residue of the second building block sequence was substituted one by one by a cysteine residue. B) Working example with anti-hFSH monoclonal antibody-02 . C). Three dimensional model of hFSH. The identified epitope is shown in black.

Fig. 5. Working example of coupling of a long peptide (24-mer) that is recognized by anti-hFSH monoclonal antibody-01 to all overlapping 15-mers covering hFSH. The example illustrates that all peptides were coupled.

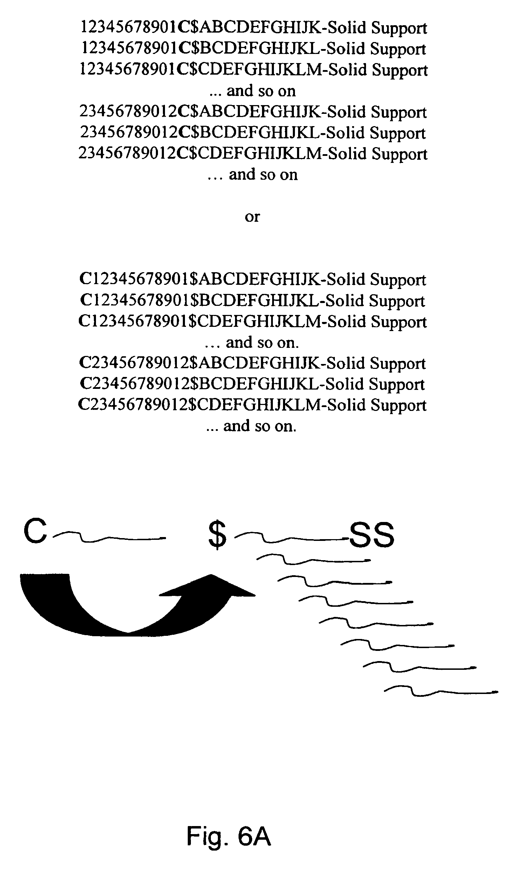

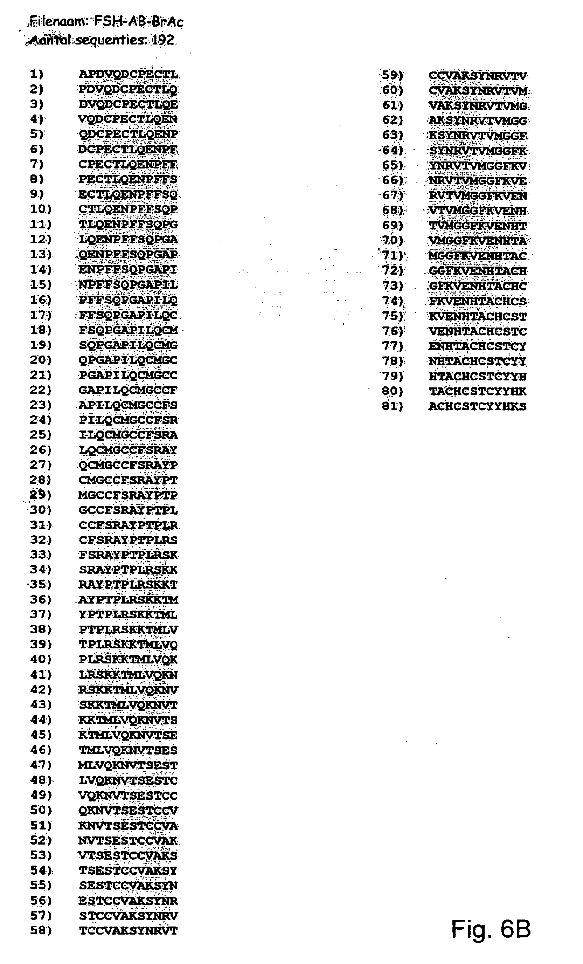

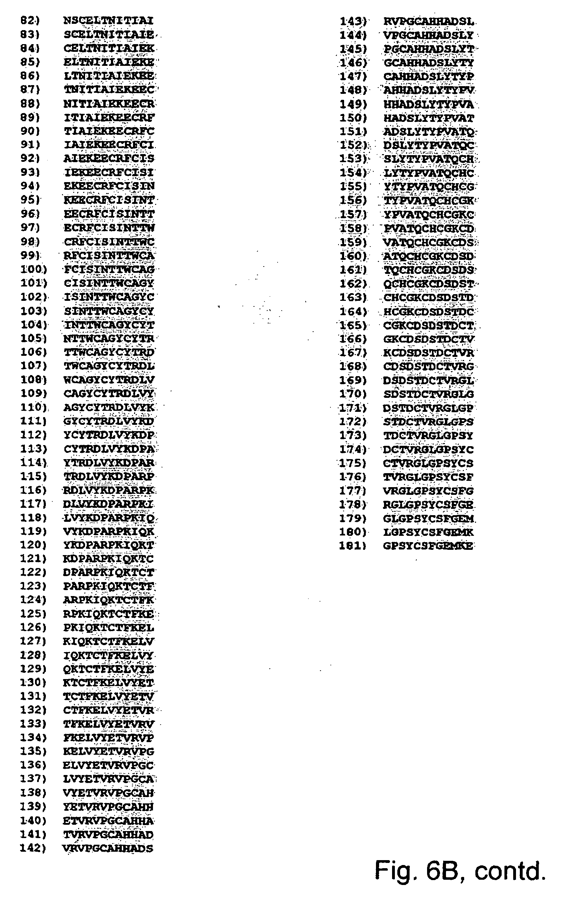

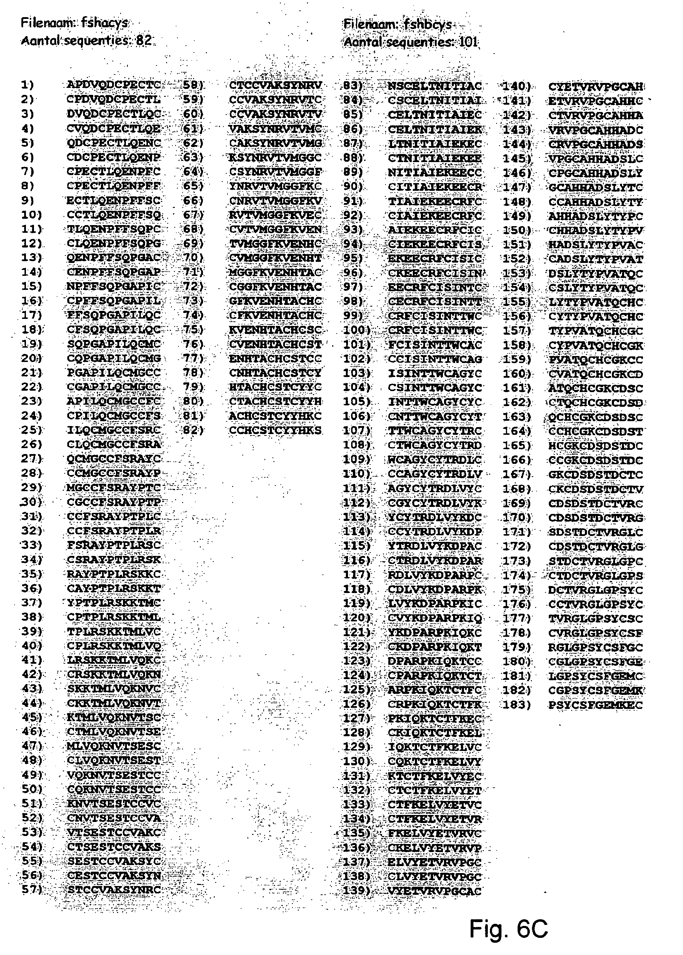

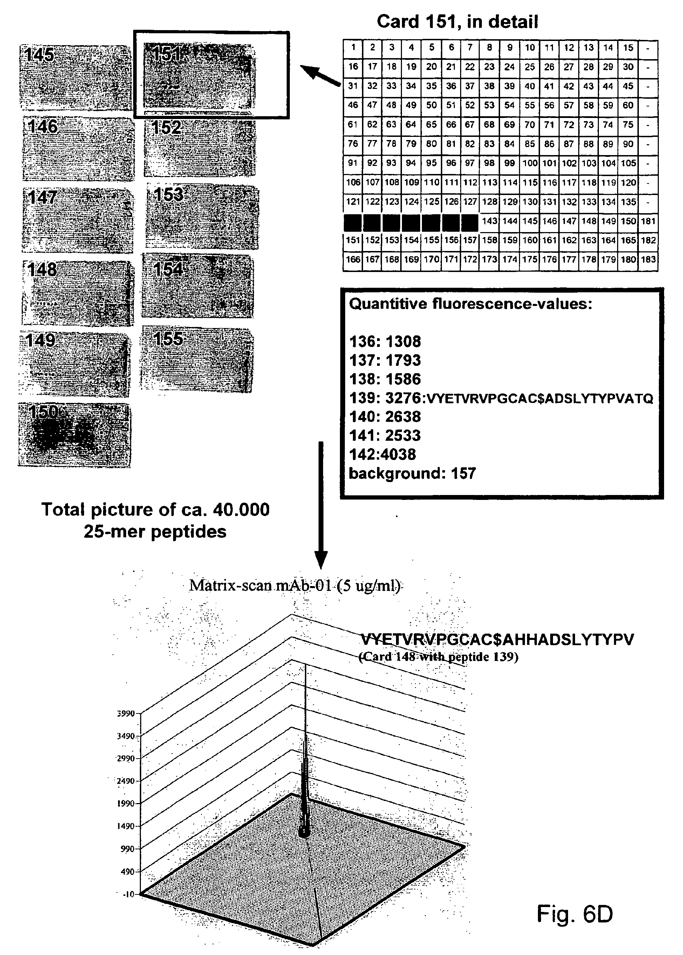

Fig. 6. A) Schematic presentation of a head-to-tail complete matrix scan. 12345678901 and ABCDEFGHIJK represent sequences derived from a protein, or Schematic presentation of a tail-to-tail complete matrix scan. In this case the cysteine residue is positioned at the N-terminus of the second building block, leading to a reversed or tail-to-tail orientation of both building blocks. Both sequences are linked as described previously. In this scan both sequences are shifted independently through the complete protein sequence, generating a library of all possible sequence combinations. B) List of all overlapping 12-mer peptides (derived from hFSH) containing an N-terminal bromoacetamide group. C) List of all overlapping 11-mer peptides (derived from hFSH) with an additional C- or N-terminal cysteine. D) Complete matrix scan, i.e. after coupling of ALL listed in fig. 6B sequences to ALL listed in fig 6C sequences, exemplified by cards 145-155 and a full picture of all binding values of all ca. 40.000 peptides (below). The best binding peptide is shown.

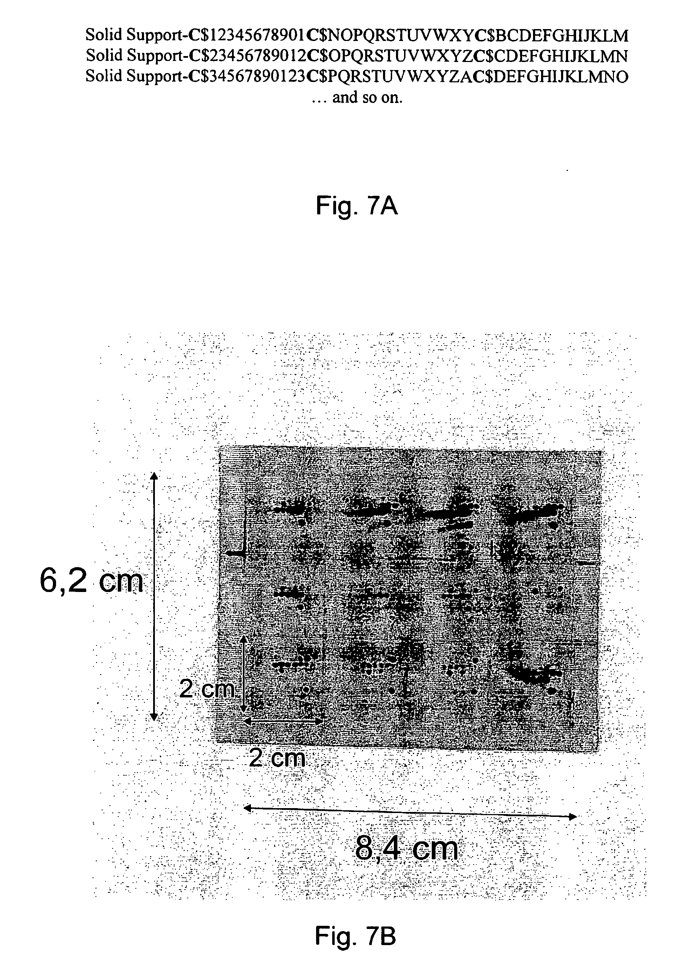

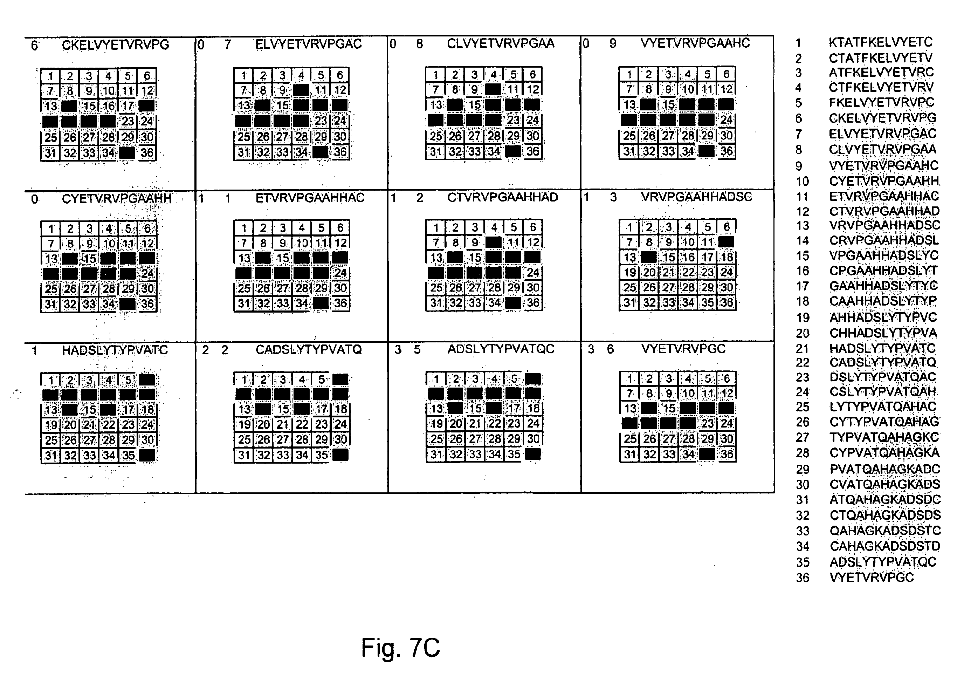

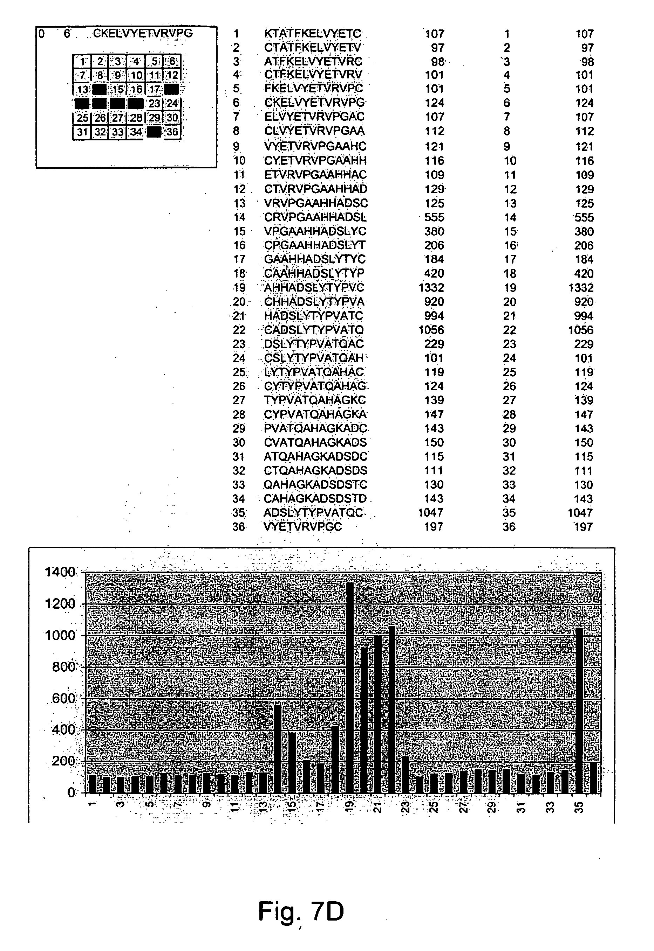

Fig. 7. A) Schematic presentation of a multi-building block scan. 12345678901 (building block 1), NOPQRSTUVWXY (building block 2) and BCDEFGHIJKLM (building block 3) represent successive sequences derived from a protein. Building blocks were linked via a thioether bridge, formed by reaction of a free thiol function of a C-terminal cysteine residue (C) in building block 1 and a bromoacetamide group ($) at the N-terminus of building block 2 and so on, as described in example 3. In this scan all sequences are subsequently shifted simultanuously through the complete protein sequence to obtain the complete library. B) Working example of obtained wityh anti-hFSH monoclonal antibody-02. C) Binding values and list of peptides coupled onto each other. D)One square in full detail. The peptide br-CKELVYETVRVPG was coupled to the cysteine of card 06. To this card peptides 1 to 36 were spotted with gridding pins. The binding values are shown below. ***Chemistry in short:*** Polypropylene (PP) surface was gamma irradiated (in this case 50kGy) in the presence of CuSO4 and in this case 12% acrylic acid. Carboxylic acid functionalized PP was treated with Boc-HMDA/DCC/HOBt and subsequent treatment with trifluoracetic acid (TFA) yielded a surface with amino groups. To this amino group functionalized PP surface, N-Fmoc-S-trityl-L-cysteine (Fmoc-Cys-(Trt)-OH) was coupled using DCC and HOBt. Subsequently the Fmoc group was removed, followed by acetylation of aminogroup. Treatment of the surface with TFA (with triethylsilan and water as scavengers) yielded a thiol functionalized surface. Bromoacetyl (or other thiol reactive) containing peptides were allowed to react with the thiol groups of the PPsurface in 0.015M NaHC03 (pH 7-8, overnight reaction). Subsequently the -StBu groups (of the S-t-butylthio protected Cys residues) of the coupled peptides were removed using NaBH4 (14mg/ml in 0.015M NaHCO3 pH 7-8, 30min, 30oC), generating new thiol groups in the peptides. A second set of Bromoacetyl (or other thiol reactive) containing peptides were then allowed to couple to the first set, making peptide constructs. This proces can be repeated several times using different sets of bromoacetylated peptides.

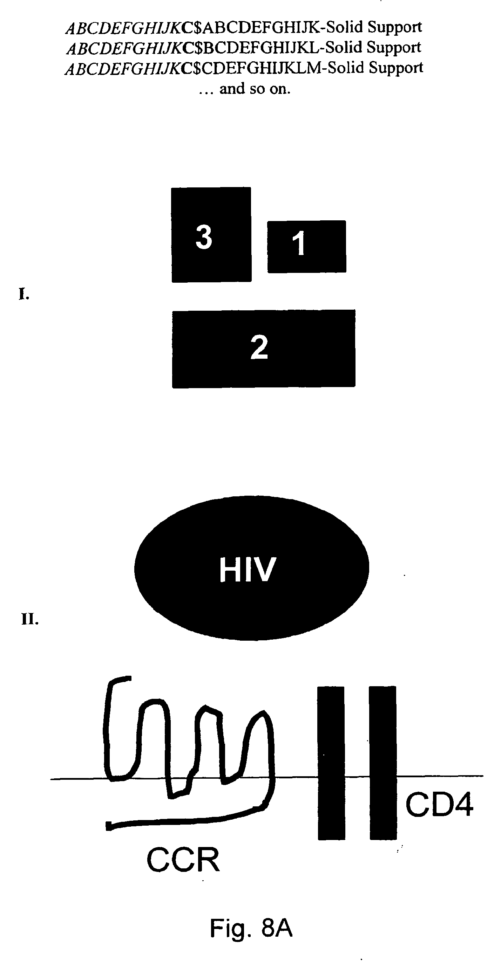

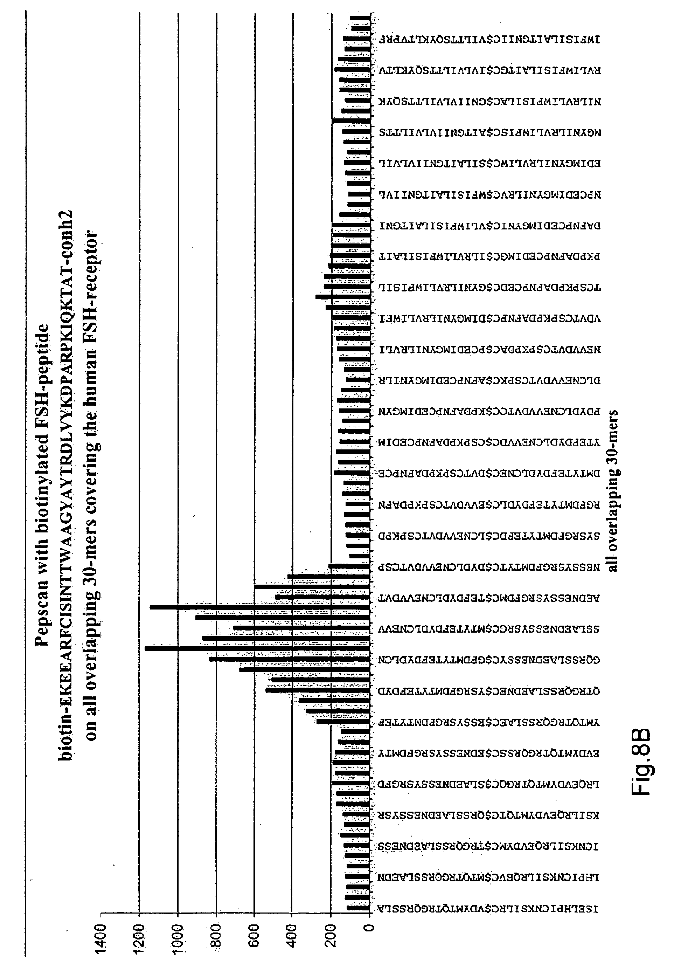

Fig. 8. A) Schematic presentation of a scan of interacting proteins 1, 2 and 3. *ABCDEFGHIJK* and ABCDEFGHIJK represent two independent sequences from two different proteins combined in one construct. **I:** a matrix scan of these building blocks was tested against a complete, labeled third protein. **II:** a matrix scan of building blocks from HIV proteins and the CCR5 protein was tested against a labeled soluble CD4 protein. B) Working example in minicards with parts of hormones and/or receptor. Shown is an example with biotinylated hFSH derived peptides on all overlapping 30-mers covering the hFSH-receptor.

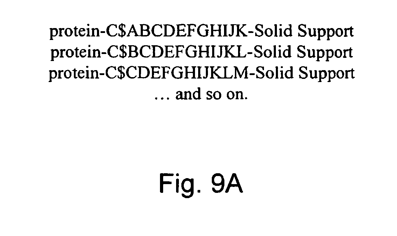

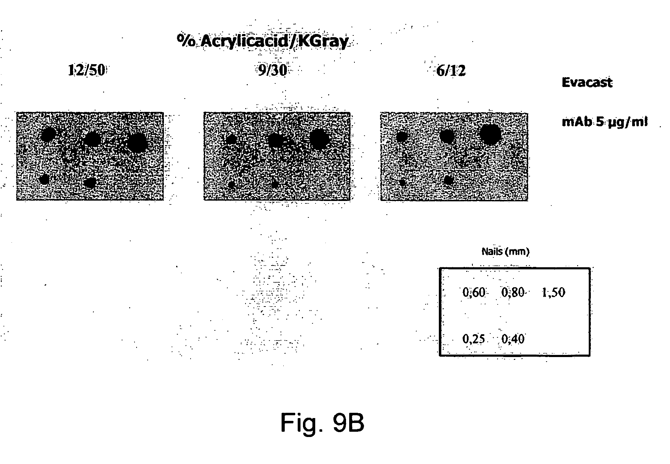

Fig. 9. A) Schematic presentation of a matrix combi-scan with a complete protein. The constructs are scanned with a another labeled protein in solution. B) Working example with whole protein glucose oxidase coupled to cysteine on surface. The protein was detected with an anti-glucose oxidase monoclonal antibody. The diameter of the gridding pins used is also shown.

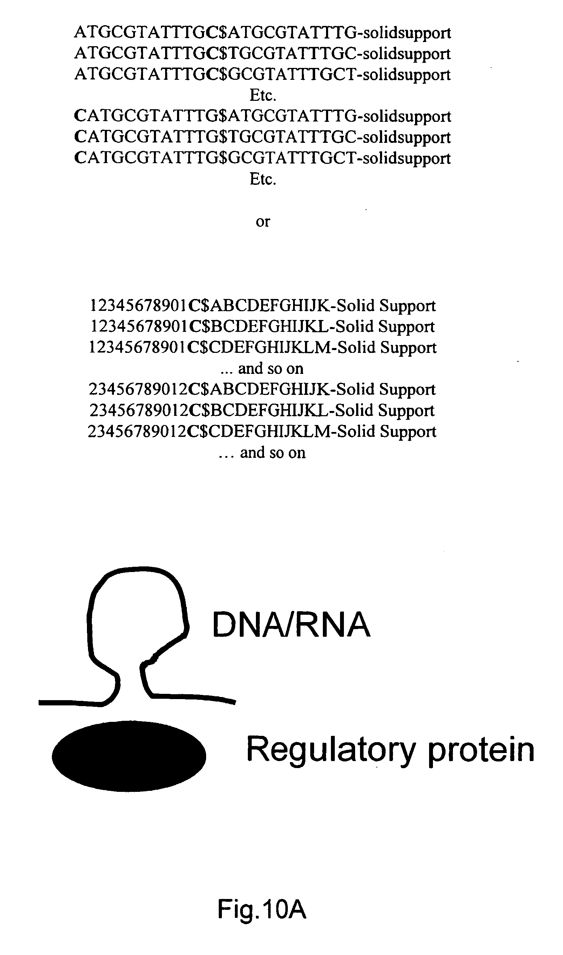

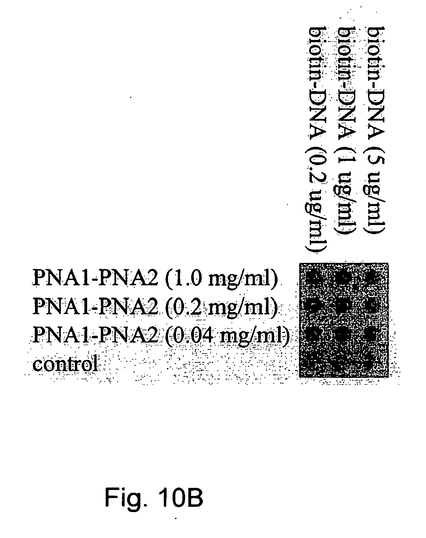

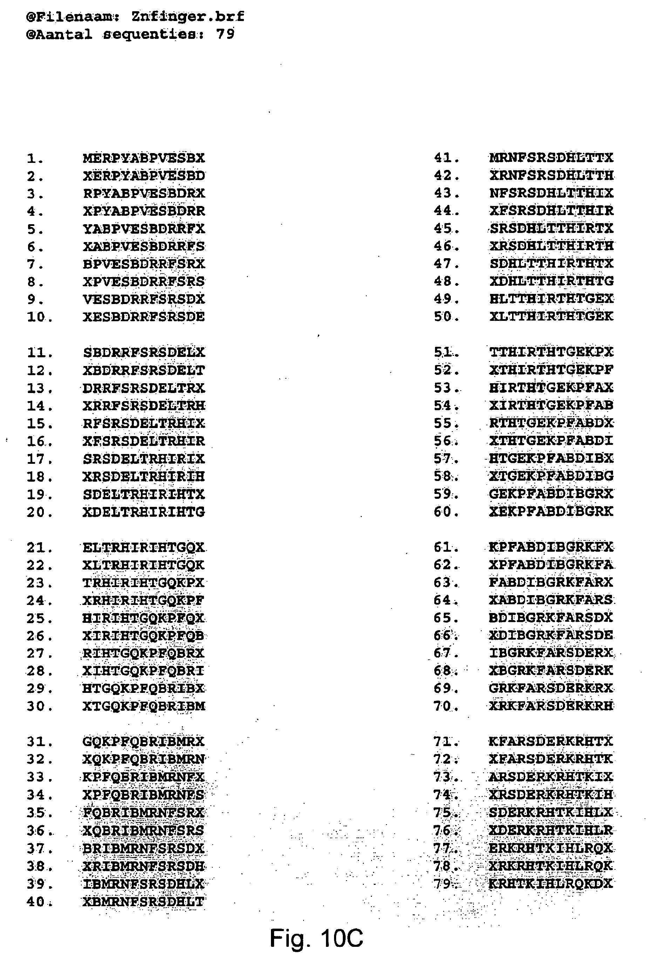

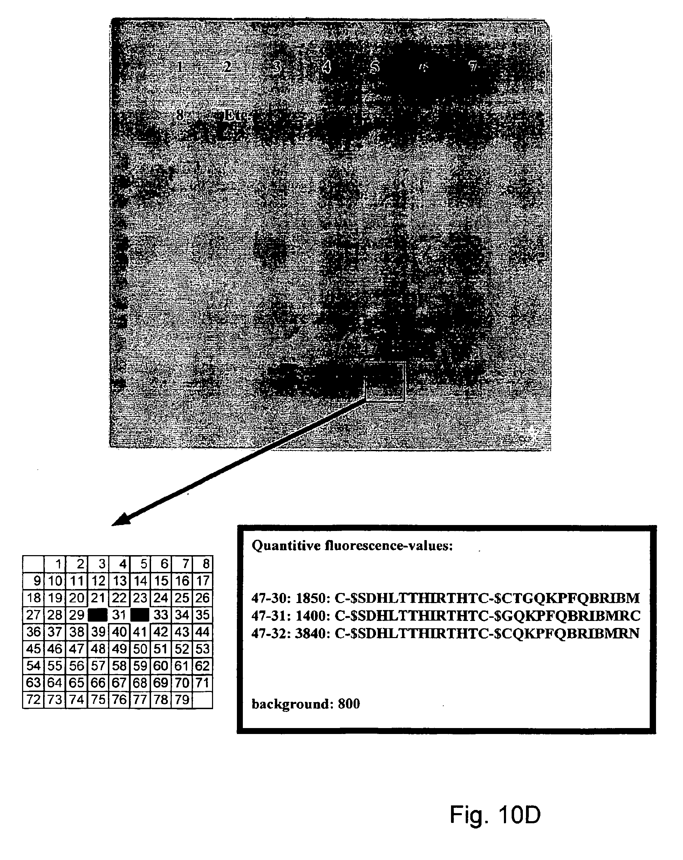

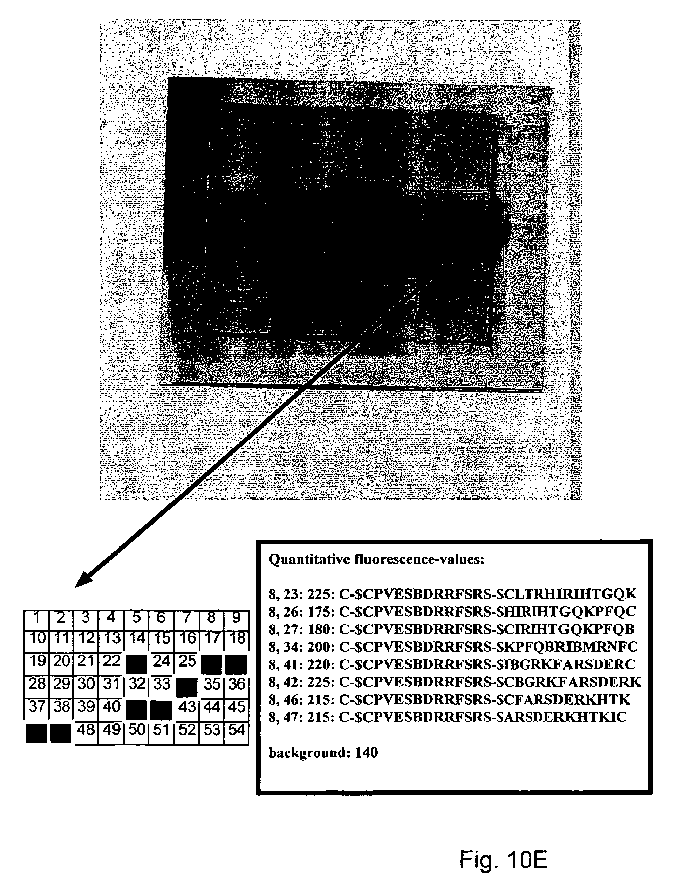

Fig. 10. A) Schematic presentation of a DNA/RNA scan. The constructs are scanned with a labeled protein, for example a regulatory protein, or another DNA or PNA strand (top), or alternatively overlapping peptides are scanned with biotynilated RNA, DNA or PNA (bottom). B) Working example with biotinylated DNA on construct of two PNA's.
***Chemistry in short:*** Detection of the binding of Biotinylated DNA by the construct of two PNAs. In miniwell setup the polypropylene (PP) surface of the miniwells was functionalized with carboxyl groups using gamma irradiation (12kGy) in the presence of CuSO4 and 6% acrylic acid. Subsequently the PP surface was amino group functionalized (using BocHMDA/DCC/HOBt with subsequent removal of the Boc group with TFA). Next the amino groups were converted in thiol groups by coupling of Fmoc-Cys-(Trt) using DCC/HOBt, removal of the Fmoc group, acetylation of the amino group followed by removal of the Trt by TFA/ triethyl silan. To this PP surface functionalized with thiol groups a PNA (PNA1) was coupled which contains N-terminal a bromoacetyl group and C-terminal an extra Cys-S-tBu (the Bromo group of the PNA reacts to the thiol group of the surface). After removal of the S-tBu (using NaBH4) of the Cys-S-tBu, the couped PNA has a thiol group on C-terminal end. A second PNA (PNA2) containing a Bromoacetyl group N-terminal is coupled to the first PNA (bromogroup of PNA2 react to thiol group of PNA1. PNA1=Br GAGGCCTGCT-Cys-S-tBu, PNA2=Br-ATGGCACTTC. In this way on the PP surface the construct GAGGCCTGCTspacer ATGGCACTTC is made. The spacer between PNA1 and PNA2 has approximately the length of one PNA-nucleotide. Figure 10B shows the binding of a biotinylated DNA (3'-TATTCTCCGGACGAGTACCGTGAAGGGTC-Biotin-5') to the PNA construct. Bound Biotinyled-DNA was detected using Streptavidin conjugated to horse radish peroxidase and ABTS. C and D) Working example of scan with biotinylated PNA on overlapping peptides illustrated by a list of peptides derived from a zinc-finger. Polypropylene surface was gamma irradiated (50 kGy) in the presence of 12% acrylic acid. Zinc-finger matrix was tested for the binding with Dna: The Zinc-finger matrix was incubated (in the presence of 0, 1mg/ml herringsperm DNA) overnight with 5'Biotin-AGCGTGGGCGT-3' hybridised to 3-'Biotin-CGCACCCGCAT-5' (5 ug/ml). After rinsing the matrix was treated with Streptavidin conjugated to alkalin-phosphatase (in the presence of 1% Bovine Serum Albumin). Rinsing and a subsequent treatment of the matrix with Vistra ECF (as described) visualized the binding between Dna and the peptide constructs of the Zinc-finger. Binding of with 5'Biotin-AGCGTGGGCGT-3' to peptides zinc-finger. (B, 4-aminobutyric acid, is a replacement for cysteine residue). E) Working example of scan with biotinylated PNA on overlapping peptides illustrated by a list of peptides derived from a zinc-finger. Polypropylene surface was gamma irradiated (12 kGy) in the presence of 6% acrylic acid. Zinc-finger matrix was tested for the binding with Dna: The Zinc-finger matrix was incubated (in the presence of 0,1mg/ml herringsperm DNA) overnight with 5'Biotin-AGCGTGGGCGT-3' hybridised to 3-'Biotin-CGCACCCGCAT-5' (20 ug/ml). After rinsing the matrix was treated with Streptavidin conjugated to alkalin-phosphatase (in the presence of 1% Bovine Serum Albumin). Rinsing and a subsequent treatment of the matrix with Vistra ECF (as described) visualized the binding between Dna and the peptide constructs of the Zinc-finger. Binding of with 5'Biotin-AGCGTGGGCGT-3' to peptides zinc-finger. (B, 4-aminobutyric acid, is a replacement for cysteine residue).

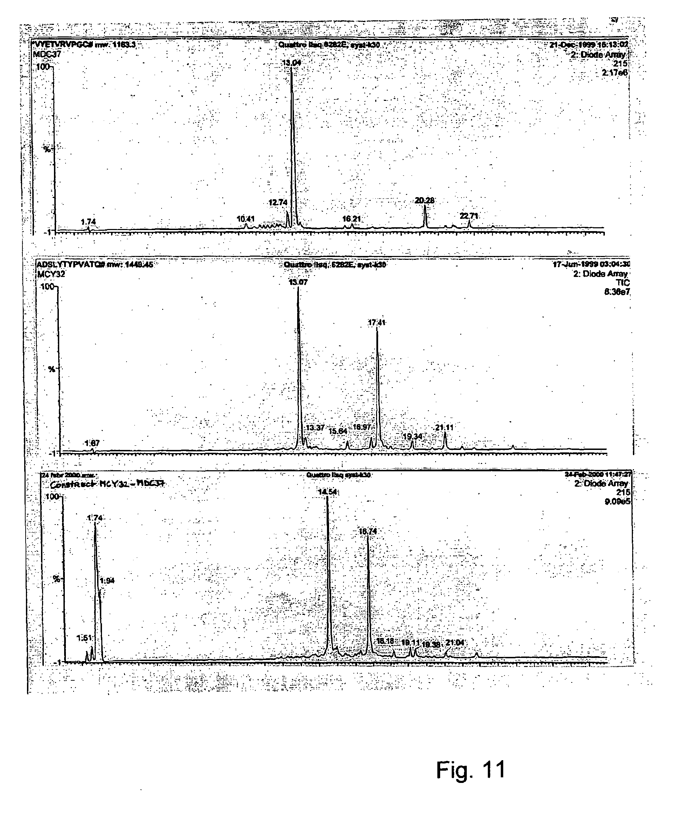

Fig. 11. Illustration of coupling bromoacetamide and cysteine in solution. Graphs shows Mass analysis of peptides before and after coupling showing that both peptides were linked into one longer peptide: A solution of 1mg/ml (in 0.03M NaHCO3) of a Bromogroup containing peptide BrADSLYTYPVATQamide was added to a 1mg/ml (in H2O) solution of a thiol containing peptide AcetylVYETVRVPGCamide. The reacting was monitored using Ellmansreagent (determines free thiolgroups). The reaction was complete within 2.5 hours. HPLC analasis reveal the product AcetyIVYETVRVPGCamide-(thioether)-ADSLYTYPVATQamide as determined with MS-Quattro.

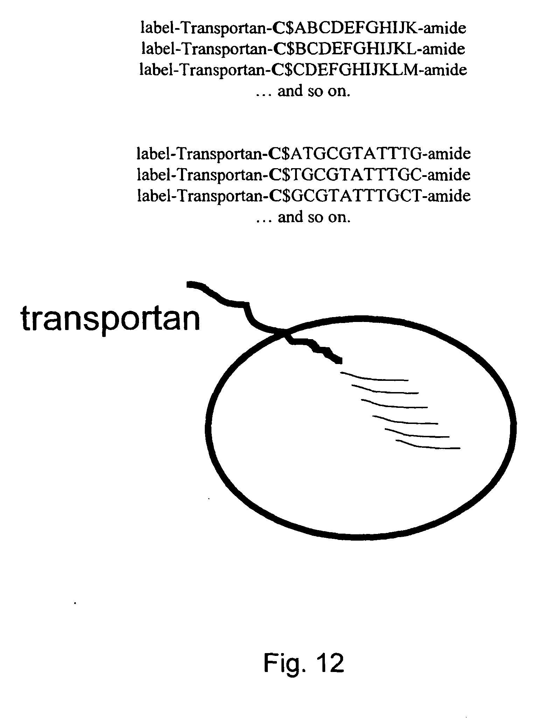

Fig. 12. Schematic presentation of a intracellular protein scan.

### Detailed description

### SYNTHESIS OF PEPTIDE CONSTRUCTS

A peptide with a N-terminal bromoacetamide group was synthesised at the surface of a solid support containing free amino groups. The peptide still contained the side-chain protecting groups of the amino acid residues. A second peptide containing a cysteine residue, which was deprotected and cleaved from another solid support was reacted with the bromoacetamide peptide on the first solid support. The formed construct was deprotected, but not cleaved from the support, and could be used in ELISA.

A polypropylene or polyethylene support, or of other suitable material, was grafted with, for instance, polyacrylic acid. As an example: a polypropylene support in a 6 % acrylic acid solution in water, containing CuSO₄, was irradiated using gamma radiation at a dose of 12 kGy. The grafted solid support containing carboxylic acid groups was functionalised with amino groups via coupling of t-butyloxycarbonyl-hexamethylenediamine (Boc-HMDA) using dicyclohexylcarbodiimide (DCC) with N-hydroxybenzotriazole (HOBt) and subsequent cleavage of the Boc groups using trifluoroacetic acid.

Standard Fmoc peptide synthesis was used to synthesise peptides on the amino functionalised solid support. After cleavage of the Fmoc group of the last amino acid and washing, bromoacetic acid was coupled using DCC or- DCC/HOBt. If only DCC was used the peptide did contain a thiol reactive bromoacetamide group, however, if DCC/HOBt was used to couple bromoacetic acid, the peptide essentially did not contain the bromo group, but another reactive group capable to react efficiently with thiol groups thus forming the same thioether link between the segments. Coupling/ligation of a second peptide to a peptide synthesised on a solid support: Peptides were synthesised at polyethylene pins grafted with polyhydromethylmethacrylate (poly-HEMA). This graft polymer was made by gamma irradiation of polyethylene pins in a 20% HEMA solution in methanol/water 80/20 or 70/30 at a dose of 30-50 kGy. The functionalised support can be used for the synthesis of 1 µmol of peptide/cm² after coupling of β-alanine and an acid labile Fmoc-2,4-dimethoxy-4'-(carboxymethyloxy)-benzhydrylamine (Rink) linker. The peptides were synthesised using standard Fmoc chemistry and the peptide was deprotected and cleaved from the resin using trifluoroacetic acid with scavengers.

The cleaved peptide containing a cysteine residue at a concentration of about 1 mg/ml was reacted with the solid support bound peptide described above in a water/sodium bicarbonate buffer at about pH 7-8, thus forming a partially protected construct of two peptides covalently bound via a thioether and C-terminally bound to the solid support.

The construct described above was deprotected following standard procedures using trifluoroacetic acid/scavenger combinations. The deprotected constructs on the solid support were extensively washed using disrupting buffers, containing sodium dodecylsulphate and β-mercaptoethanol, and ultrasonic cleaning and were used directly in ELISA. Subsequent cleaning in the disrupt buffers allows repeatingly testing against other antibodies in ELISA.

Figure 1 shows an example of the ELISA results of screening a simple library of constructs, consisting of a dodecapeptide segment coupled via its C-terminally added cysteine residue to a N-terminally bromoacetylated second segment, scanning a protein sequence by steps of a single amino acid residue. The bromoacetamide peptide was covalently bound to a functionalised polypropylene/polyacrylic acid solid support in 3 µl wells as described above. The cysteine-containing sequences were synthesised on and cleaved from functionalised polyethylene pins as described above. As shown in figure 1, high binding was observed in ELISA for constructs around position 125, which consists of the segments [125-136] and [139-150], linked via a thioether bond. A conventional linear PEPSCAN of dodecapeptides or 15-peptides did not show any binding in a reaction against the same monoclonal antibody.

On a surface of a solid support peptides are synthesized with a bromoacetamide group at the N-terminus as described above. On this peptide functionalized support a second peptide segment containing a free thiol group was spotted using piezo drop-on-demand technology, using a microdosing apparatus and piezo autopipette (Auto Drop-Micropipette AD-K-501) (Microdrop Gesellschaft fur Mikrodosier Systeme GmbH. Alternatively, spotting or gridding was done using miniature solenoid valves (INKX 0502600A; the Ice Company) or hardened precision ground gridding pins (Genomic Solutions, diameters 0.4, 0.6, 0.8 or 1.5 mm). Subsequent deprotection of the construct and extensive washing to remove uncoupled peptide gave dipeptide constructs at the spotted area.

Figure 2 shows binding of the same antibody as was tested in figure 1 with constructs consisting of two peptide segments, generated with different volumes of spotted peptides 2 to 8, ranging from 1 µl - 0.25 nl (x-axis). Within the square the whole surface was covered with peptide 1, which was synthesised directly on this surface, only the spots contain constructs. The y-axis shows different constructs, consisting of peptide 1 with peptide 2 up to 8. Peptides 2 up to 8 are overlapping dodecapeptides, while peptide 1 is sequence [139-150] of the same protein as described in figure 1.

Figure 2 shows that peptide constructs generated with peptide solution droplets in the nanoliter-range, bind enough antibody for detection, in this case using indirect fluorescence detection. Spots generated with 0.25 nl - 50 nl are smaller than 1 mm². Thus, peptide construct density can be as large as 100-1000 spots per cm².

### Examples of use

Proteins and peptides can be screened using for instance antibodies, soluble receptors, which contain a Fc-tail or a tag for detection, biotinylated molecules or fluorescent molecules. Alternative building blocks could be, for instance, carbohydrates, non-natural amino acids, PNA's, DNA's, lipids, molecules containing peptide bond mimetics. In the examples $ is used as a symbol for the thioether link formed by reaction of the thiol group of a cysteine residue of one building block with a bromoacetamide at the N-terminus or at the side chain of a lysine residue from another building block. This symbol can also be used for other linking chemistries as described.

The examples are divided into two types. Type I is performed in the creditcard format minicards (cf. Fig. 1). Type II is performed using gridding pins on a discontinuous porous matrix surface (cf. Fig. 2). For each example the type is indicated between brackets.

### Example-1 (type I): 'standard' 24-30-mer scan of linear sequence, containing two building blocks.

In this example the consecutive sequences of the building blocks are both shifted one by one residue through the sequence of the protein to be tested as shown in Fig. 3A and exemplified in Fig. 3B and 3C for 30-mers (in Fig. 1 the example is with 24-mers). The -C$- link between both building blocks replaces 0 - 2 or more amino acid residues of the native protein sequence. Applications include replacement sets of peptides, in which amino acid residues are replaced systematically by other amino acid residues (Fig. 1C), deletion sets of peptides, in which amino acid residues are deleted systematically, and combination sets, in which peptides of different lenght ranging from 2 - 24 (here building block 2, 2-40 or more and building block 1, 2-15 or more) amino acid residues are used.

### Example-2a (type I): Positional scan with cysteine at different positions.

This is a scan similar to example 1 described above, however, in this scan the cysteine is used to substitute the amino acid residues one by one in every position of the second building block as shown in Fig. 4A and exemplified in Fig. 4B and 4C. Fig. 5 illustrates the reproducibility of coupling a 25-mer that binds mAb-01 to all overlapping 15-mers.

### Example-2b (type II): head-to-tail matrix-scan.

In type-I, i.e. using the creditcard sized minicards only a few thousand peptides can be synthesized. In type-II, i.e. using the gridding pins, many thousands of peptides (in the order of 40.000) can be synthesized simultaneously.

In a complete matrix-scan the N-terminal sequence of, for instance, sequence [1 - 11] of a protein, is linked as a building block with each overlapping peptide sequence of a complete scan of the same protein as shown in figure 6A. Next, sequence [2 - 12] is linked with the same set of overlapping sequences and so on. The link can be formed, for instance, by reaction of a cysteine at the C-terminus of the second building block with a bromoacetamide modified N-terminus of the first building block. This means that every combination of, for instance, undecapeptides from the protein sequence is being synthesised on a seperate, known, position of the solid support.

### Example-2c (type II): tail-to-tail matrix-scan.

This is the same scan as the complete matrix scan from example 2a, however, in this scan the cysteine of the second building block is located at its N-terminus, providing a reversed or tail-to-tail orientation of both building blocks in the construct as also shown in figure 6A.

Both example 2b and 2c are illustrated in Figs 6B, 6C and 6D.

### Example-3(type-II): Multi building block scan.

In this example a thiol fuction is introduced on an amino-functionalised solid support. This can be made by a direct reaction of the amino groups with, for instance, iminothiolane, or by coupling of Fmoc-Cys(Trt)-OH, followed by Fmoc cleavage using piperidine, acetylation, and trityl deprotection using TFA/scavenger mixtures. This thiol-functionalised solid support can be reacted with, for instance, a bromoacetamide-peptide, containing a protected cysteine residue. After coupling of the first peptide, the cysteine can be deprotected, using, for instance, a TFA/scavenger mixture. The formed free thiol group can be used to couple a second bromoacetamide-peptide, again containing a protected cysteine. This procedure can be repeated to make multi-building block constructs. Several types of scans, as described in the other examples, can be used in combination with this multi building block scan. In fig. 7A an example is shown for a three multi building block scan. An working example with two building block scan is illustrated in 7B, 7C and 7D.

### Example-4 (type-I): Matrix combi-scan, interaction between three (or more) proteins

In a matrix combi-scan, a matrix scan from two different proteins is tested against a labeled soluble protein. Figure 8A shows two examples. In the first example (fig. 8A) soluble protein 1 (growth hormone, GH) was tested against a combined matrix scan of protein 2 (GH-receptor-1) and protein-3 (GH-receptor-2). In the second example a part of soluble protein 1 (CD-4) was tested against a combined matrix scan of protein 2 (HIV) and protein 3 (chemokine-receptor CCR4). In Fig. 8B the usage of receptors and hormones is illustrated by using a biotin-labeled part of the protein human Follicle Stimulating Hormone tested on all overlapping 30-mers covering te human human Follicle Stimulating Hormone receptor.

Examples 1 to 4 describe methods using peptide building blocks and screening with proteins. These constructs can also be screened against non-proteins. Also non-peptide building blocks can be used. Below, examples of whole proteins in combination with peptides (example 5), or peptides/proteins in combination with non-peptide/proteins, or non-peptide/protein with non-peptide/protein (example 6, DNA/RNA/PNA) are shown.

### Example-5 (type II): Matrix combi-scan, interaction between three (or more) proteins

This example is similar to example 4. The difference is that the building block 2 sequences, derived from one protein (*ABCDEFGHIJKC* etc.) are replaced by a complete protein, which contains an added thiol group for coupling (see Fig. 9A). To illustrate that native proteins can be used to be coupled in this way the protein glucose oxidase was used as an example (Fig. 9B).

### Example-6 (type I and type-II): scans of linear DNA/PNA/peptide with DNA/PNA/peptide

This example is similar to that of examples 1 to 5 with the difference that one or more other non-peptide building blocks are used (DNA, RNA or a peptide nucleic acid (PNA) instead of a peptide building block). The nucleotide building blocks or PNA's are modified with groups that enable linking of the building blocks as in examples 1 to 5. Screening is performed with labeled DNA strands, peptides or proteins (see fig 10). As alternative labeled DNA or PNA strands can also be tested against peptide construct described in examples 1 to 5. The binding binding between peptide and PNA is illustrated in Figs 10B and 10C, 10D.

In addition to scanning interaction regions of proteins and non-proteins (DNA/RNA) in ELISA, chip or or blot format it is also possible to use to -C$- coupling in in vitro bio-assays. Firstly, it is possible to use soluble constructs as explained in example-3 as potential (ant)agonists for membrane bound receptors. Secondly, it is possible to use membrane-transporting proteins such as transportan or penetratin to get any of the above mentioned combinations of peptides or peptides with PNA or peptides with (small) proteins into the cell.
In Fig.11 it is illustrated that it is possible to couple two peptides in solution. In this example peptides similar to these shown in for example Figs 1A, 1B, 2B and 3B.

### Example-7a (type I): Intracellulair protein scan coupled to membrane-penetrating Transportan.

An intracellulair protein, like a kinase, can be scanned using overlapping peptides on a solid support, containing a C-terminal cleavable linker. The peptides were synthesised with a N-terminal bromoacetamide group. Next, a membrane penetrating transportan peptide, containing a label and a thiol group was coupled with the sequences. These constructs were selectively cleaved from the solid support and tested in a bioassay. Labels that can be used are, for instance, biotine or fluorescent labels (Fig. 12).

### Example-7b (type I): Intracellulair RNA, DNA or PNA-scan coupled to membrane-penetrating Transportan.

To identify PNA/DNA sequences that can be used to block expression of a particular gene a long linear scan, coupled to membrane penetrating peptide, can be tested in an in vitro bio-assay (Fig. 12).

## Claims

1. A method for producing a plurality of molecules for identification or detection of a binding site comprising generating at least one of said molecules by at least linking a first nucleic acid or peptide to a second nucleic acid or peptide, wherein said first and second nucleic acid or peptide comprise at least a trinucleotide or tripeptide.

2. A method according to claim 1, wherein a first peptide is linked to a second peptide.

3. A method according to any one of claims 1 to 2 wherein a first peptide is linked by a thioether bond to a second peptide.

4. A method according to any one of claims 1 to 3 wherein each of at least a first and/or a second nucleic acid or peptide or part thereof represents a potential part of a discontinuous binding site.

5. A method according to any one of claims 1-4, further comprising screening the obtained plurality of molecules with at least one potential binding molecule and detecting binding between a member of said plurality of molecules and said potential binding molecule.

## Patentansprüche

1. Verfahren zur Herstellung einer Mehrzahl von Molekülen zur Identifikation oder Detektion einer Bindungsstelle, welches das Erzeugen zumindest eines der Moleküle durch Binden zumindest einer ersten Nukleinsäure oder eines ersten Peptids an eine zweite Nukleinsäure oder ein zweites Peptid umfasst, wobei die erste und die zweite Nukleinsäure oder das erste und das zweite Peptid zumindest ein Trinukleotid oder Tripeptid umfasst.

2. Verfahren nach Anspruch 1, bei welchem ein erstes Peptid an ein zweites Peptid gebunden wird.

3. Verfahren nach einem der Ansprüche 1 bis 2, bei welchem ein erstes Peptid durch eine Thioetherbindung an ein zweites Peptid gebunden wird.

4. Verfahren nach einem der Ansprüche 1 bis 3, bei welchem zumindest eine erste und/oder eine zweite Nukleinsäure oder ein erstes und/oder zweites Peptid oder Teil einer/eines solchen jeweils einen potentiellen Teil einer diskontinuierlichen Bindungsstelle darstellt.

5. Verfahren nach einem der Ansprüche 1 bis 4, welches ferner das Überprüfen der erhaltenen Mehrzahl von Molekülen mit zumindest einem potentiell bindenden Molekül und das Erkennen einer Bindung zwischen einem Element der Mehrzahl von Molekülen und dem potentiell bindenden Molekül umfasst.

## Revendications

1. Procédé destiné à produire une pluralité de molécules pour l'identification ou la détection d'un site de liaison consistant à engendrer au moins une desdites molécules au moins en liant un premier acide nucléique ou peptide à un second acide nucléique ou peptide, lesdits premier et second acides nucléiques ou peptides comprenant au moins un trinucléotide ou tripeptide.

2. Procédé selon la revendication 1, dans lequel un premier peptide est lié à un second peptide.

3. Procédé selon l'une quelconque des revendications 1 à 2, dans lequel un premier peptide est lié par une liaison thioéther à un second peptide.

4. Procédé selon l'une quelconque des revendications 1 à 3, dans lequel chacun d'au moins un premier et/ou un second acide nucléique ou peptide ou une partie de celui-ci représente une partie potentielle d'un site de liaison discontinu.

5. Procédé selon l'une quelconque des revendications 1 à 4, consistant en outre à cribler la pluralité de molécules obtenue avec au moins une molécule de liaison potentielle et à détecter la liaison entre un membre de ladite pluralité de molécules et ladite molécule de liaison potentielle.
